(19) Europäisches Patentamt
European Patent Office
Office européen des brevets

(11) Publication number : **0 430 645 B1**

# (12) EUROPEAN PATENT SPECIFICATION

(45) Date of publication of patent specification : **18.08.93 Bulletin 93/33**

(51) Int. Cl.$^5$ : **C12N 15/31,** C12N 15/81, C12P 21/00, A61K 39/08

(21) Application number : **90312870.0**

(22) Date of filing : **27.11.90**

(54) **Vaccines.**

(30) Priority : **28.11.89 GB 8926832**
**17.03.90 GB 9006097**

(43) Date of publication of application : **05.06.91 Bulletin 91/23**

(45) Publication of the grant of the patent : **18.08.93 Bulletin 93/33**

(84) Designated Contracting States : **AT BE CH DE DK ES FR GB IT LI LU NL SE**

(56) References cited :
**FR-A- 2 249 679**
**BIOTECHNOLOGY, vol. 7, October 1989, pages 1043-1046; A.J. MAKOFF et al.: "Expression of tetanus toxin fragment c in E. Coli: Its purification and potential use as a vaccine"**
**EUR. J. BIOCHEM., vol. 182, February 1989, pages 649-656; U. WELLER et al.: "Chains and fragments of tetanus toxin separation, reassociation and pharmacological properties"**
**NUCLEIC ACIDS RESEARCH, vol. 14, no. 19, 1986, pages 7809-7812, IRL Press Ltd, Oxford, GB; N.F. FAIRWEATHER et al.: "The complete nucleotide sequence of tetanus toxin"**
**PROC. NATL. ACAD. SCI. USA, vol 86, June 1989, pages 4097-4101; B.J. OSBORNE et al.: "Mutational analysis of a yeast transcriptional terminator"**

(56) References cited :
**BIOTECHNOLOGY AND GENETIC ENGINEERING REVIEWS, vol. 3, September 1985, pages 377-416, Intercept Ltd; S.M. KINGSMAN et al.: "Heterologous gene expression in saccharomyces cerevisiae"**
**THE JOURNAL OF BIOLOGICAL CHEMISTRY, vol. 257, no. 6, 25th March 1982, pages 3018-3025; J.L. BENNETZEN et al.: "The primary structure of the saccharomyces cerevisiae gene for alcohol dehydrogenase I"**

(73) Proprietor : **THE WELLCOME FOUNDATION LIMITED**
**Unicorn House 160 Euston Road**
**London NW1 2BP (GB)**

(72) Inventor : **Makoff, Andrew Joseph, The Wellcome Research Lab.**
**Langley Court**
**Beckenham, Kent, BR3 3BS (GB)**
Inventor : **Romanos, Michael Anthony**
**The Wellcome Research Lab. Langley Court**
**Beckenham, Kent, BR3 3BS (GB)**
Inventor : **Clare, Jeffrey John, The Wellcome Research Lab.**
**Langley Court**
**Beckenham, Kent, BR3 3BS (GB)**
Inventor : **Fairweather, Neil Fraser**
**The Wellcome Research Lab. & Langley Court**
**Beckenham, Kent, BR3 3BS (GB)**

(74) Representative : **Stott, Michael John et al**
**The Wellcome Foundation Limited Group Patents & Agreements Langley Court**
**Beckenham Kent BR3 3BS (GB)**

**EP 0 430 645 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the production of tetanus toxin C fragment.

Vaccination against tetanus is effective in the prevention of this disease in most Western countries, although incomplete vaccination in some third world countries can account for up to one million cases of tetanus every year. Current tetanus vaccines are produced by formaldehyde treatment of tetanus toxin produced by the anaerobic bacterium C. tetani to produce the immunogenic toxoid. It has been suggested that impurities incorporated during formaldehyde treatment are partly responsible for the adverse effects sometimes seen with hyperimmunisation with tetanus toxoid.

The structural gene for tetanus toxin has been cloned and sequenced (Fairweather, N.F., et al, J. Bacteriol. 165, 21-27 (1986); Fairweather, N.F., and Lyness, V.A., Nuc. Acid Res. 14, 7809-7812 (1986). These studies have confirmed the structure of tetanus toxin as a 150kD protein of 1315 amino acids. The toxin can be cleaved by various treatments into several fragments. Fragment C, comprising the C terminal 451 amino acids, is a 50kD polypeptide generated by papain cleavage of toxin. FR-A-2249679 describes the preparation of an inactivated tetanus toxin immunogen by treating tetanus toxin with a proteinase.

Fragment C derived in this way has been shown to be non-toxic and is capable of immunising mice and guinea pigs (Helting, T.B., and Zwisler, 0., J. Biol. Chem. 252, 187-193 (1977); Helting, T.B., and Nau, H.H., Act. Pathol. Microbiol. Scan. Sect. C 92, 59-63 (1984)). Papain digestion also releases the 100 kD fragment B, comprising the N-terminal part of the toxin molecule. Fragment B is also protective, but has been reported to be toxic to animals at high doses (Helting, T.B., et al, J. Biol. Chem. 253, 125-129, (1978)).

Portions of tetanus toxin containing fragment C have been expressed in E.coli (Fairweather, N.F., et al, J. Bacteriol, 165, 21-27, (1986)); Fairweather, N.F., et al, Infection and Immunity 55, 2541-2545, (1987); EP-A-0209281). These portions of tetanus toxin which were expressed were either fused to part of the E.coli trpE protein or comprised part of fragment B and all of fragment C of tetanus toxin. All the above were found to be expressed at low levels and were all insoluble in the cytoplasm of E.coli cells.

It has been found previously that when fragment C on its own is expressed in E.coli, it is soluble in the cytoplasm of the cells. Fragment C was expressed using two plasmids, pTETtac1 and pTETtac2 which were derived from the high expressing plasmid pIFGtac124A (Makoff, A.J., et al., Biochem.Soc. Trans., 16, 48-49, (1988)) Most of the coding sequence of pTETtac1 was provided by two restriction fragments. The rest of the sequence was encoded by a pair of synthetic oligonucleotides both 42 base pairs long, where the codon bias was optimised for expression in E.coli. Plasmid pTETtac2 was constructed from pTETtac1 by replacing the BglII-Sfa NI region by a pair of synthetic oligonucleotides (each 161 nucleotides long) which reproduced the sequence upstream of the initiation codon and optimised the coding sequence, at the beginning of the C fragment region, for expression in E.coli (Makoff, A.J., et al. Bio/Technology 7, 1043-1046 (1989)).

However, E.coli has the disadvantage as a host organism that it contains toxic pyrogenic factors (lipopolysaccharides from the cell wall) which must be rigorously excluded from the final product. The ease with which these factors may be excluded will depend on the protein product in question and the method by which it is purified from the cell. However, it would be preferable to eliminate the possibility of contamination altogether simply by using a non-toxic organism as the host, such as yeast.

In using the native sequence encoding fragment C, the inventors were unable to obtain expression in yeast and found that the barrier to expression was due to the fact that the mRNA transcripts of the gene were incomplete. Synthesis of the complete transcript probably involves at the 3'-end three closely linked steps: termination of the primary transcript, endonucleolytic processing and polyadenylation. (Platt, J.,Ann.Rev.Biochem., 55, 339-372, (1986)). The inventors have now identified the position of several "terminators" (termination/endo- nucleolytic processing/polyadenylation sites) present in the DNA. As a result the inventors were able to eliminate these and obtain successful expression in yeast of tetanus toxin fragment C.

Figure 1 shows the position of at least six elements which are completely or partially responsible for the production of incomplete mRNA transcripts. The yeast terminator is poorly defined. Several different consensus sequences have been proposed (Henikoff, S., et al., Cell, 33, 607-614, (1983); Zaret, K.S., and Sherman, F., Cell, 28, 563-573, (1982); Bennetzen, J.L., and Hall, B.D., J.Biol.Chem., 257, 3018-3025, (1982a)), but it appears that there may be deviation from these sequences and it appears that other, undefined elements may also be necessary for termination (Osborne, B.I., and Guarente L., PNAS, 86, 4097-4101, (1989)). Yeast terminators occur in stretches of (A+T)-rich DNA, though not all (A+T)-rich DNA contains terminators. Our surprising finding was that the original fragment C DNA contained at least six elements which were responsible for incomplete transcription of the mRNA. The elements were eliminated by increasing the (G+C)-content at these positions thus providing for the production of a substantially complete mRNA transcript.

The present invention provides a novel DNA sequence encoding tetanus toxin fragment C and having a (G+C)-content that has been increased in the region from nucleotide 410 to the 3' end of the coding sequence relative to the wild-type DNA sequence so as to allow the production of complete mNRA transcripts in yeast, the nucleotide numbering corresponding to that set forth in Figure 2 and SEQ ID NOS:1 and 2.

Tetanus toxin fragment C, as used herein, is defined as the wild type polypeptide having the amino acid sequence set forth in Figure 2 and in SEQ ID NO:1 or is a mutant polypeptide having an amino acid sequence that is at least 90% homologous with that set forth in Figure 2 and in SEQ ID NO:1 and that retains substantially the same biological and immunogenic properties as the wild-type polypeptide.

The amino acid sequence of fragment C may be varied by one or more amino acid substitutions, extensions, insertions and/or deletions provided the resulting polypeptide retains substantially the same biological and immunogenic properties as wild-type fragment C.

In the case of amino acid substitutions, one or more of the amino acid residues of fragment C may be replaced by one or more other amino acid residues which achieve this aim. Candidate substitutions include Ser for Thr and vice versa, Glu for Asp and vice versa, Gln for Asn and vice versa, Leu for Ile and vice versa, Ala for Val and vice versa and Arg for Lys and vice versa.

Mutant fragment C may be obtained by introducing nucleotide changes into the DNA sequence encoding wild-type fragment C, for example into the DNA sequence of Figure 2 and SEQ ID NO:1. This may be achieved by any appropriate technique, including restriction of the sequence with an endonuclease, insertion of oligonucleotide linkers, use of an exonuclease and/or a polymerase and site-directed mutagenesis.

Fragment C wild-type DNA has a (G+C) - content of 29%, while the preferred DNA sequence in accordance with the present invention (see Figure 2 and SEQ ID NO:2) has 47%. The maximum possible (G+C) - content that can encode fragment C is 60%. A level of 40-60% (G+C)- content would thus allow the production of a complete mRNA transcript provided that were no localised concentrations of (A+T) rich DNA.

In designing a fragment C gene for expression in yeast, one route would be to use codons found in highly expressed yeast genes (Bennetzen, J.L., and Hall, B.D., J.Biol.Chem., 257, 3026-3031, (1982)) This would increase the (G+C)-content. Another important consideration would be to eliminate runs of (A+T) since these would raise the local (A+T)-content and might be sufficient to cause termination.

Since the elements responsible for the production of incomplete transcripts are only likely to extend over apporximately 100 nucleotides, it is possible to achieve the same result by only increasing the (G+C)-content within these small regions.

Six regions were identified as being responsible for the incomplete production of mRNA transcripts by analysis of a number of different mutant DNA sequences containing differing lengths of DNA for which the (G+C)-content had been increased.

TABLE 1

| Region responsible for production of incomplete transcript in *C.tetani* DNA (nucleotides into coding sequence) | Region to be altered so as to allow the production of complete mRNA transcripts |
|---|---|
| 1. 560 ± 50 | 410 - 610 |
| 2. 660 ± 50 | 510 - 710 |
| 3. 800 ± 50 | 650 - 850 |
| 4. 1000 ± 100 | 800 - 1100 |
| 5. 1100 ± 100 | 900 - 1200 |
| 6. 1300 ± 100 | 1100 - 3' end of the coding sequence (theoretical limit of region to be altered is nucleotide 1400) |

regions 2 and 4 are most important. In order to allow the production of complete mRNA transcripts which is being prevented by regions 2 and 4 the (G+C)-content of mutant fragment DNA is increased relative to the native DNA sequence from nucleotide 510 to nucleotide 710 and from nucleotide 800 to nucleotide 1100. The next most important regions are 3, 5 and 6. Similarly, in order to allow the production of complete mRNA transcripts which are additionally being prevented by regions 3, 5 and 6 the (G+C)-content is additionally increased from nucleotide 650 to nucleotide 850, from nucleotide 900 to nucleotide 1200 and from nucleotide 1100 to the 3' end of the coding sequence. Accordingly, it is preferred that the said (G+C)-content has been increased in each of the following regions:

(i) from nucleotide 510 to nucleotide 710,
(ii) from nucleotide 650 to nucleotide 850,
(iii) from nucleotide 800 to nucleotide 1100,
(iv) from nucleotide 900 to nucleotide 1200 and
(v) from nucleotide 1100 to the 3' end of the coding sequence.

Region 1 may be too weak to interfere with the production of complete mRNA transcripts. However, in order to allow complete mRNA production which is being prevented by Region 1 the (G+C)-content is additionally increased in the region:

(vi) from nucleotide 410 to nucleotide 610.

The novel DNA sequence according to the invention may be chemically synthesised and cloned using methodologies well-known in the art. The novel DNA may then be cloned into a suitable vector and used to transform yeast which is then capable of expressing the polypeptide which is encoded by the novel DNA. The vector may be any appropriate vector which is suitable for the cloning of the DNA and which may be used to transform a yeast cell and thereby express the relevant protein. Such vectors include autonomously replicating plasmids and chromosomal integration vectors.

Vectors which may be used for cloning DNA include pWYG7 (see Example 1 and Figure 3), pWYG5 (see Example 2 and Figure 5) and pPIC3 (Example 6) for use in yeast.

In yet another feature of the present invention there is provided an expression vector, which incorporates a DNA sequence according to the invention and which is capable of expressing fragment C in yeast (See Examples 4 and 5).

The expression vector incorporates control elements for transcriptional initiation (promoters) and termination. The coding sequence of the gene to be expressed along with its own translational start and stop codons is inserted between these control elements.

Examples of promoters for use with the expression vector of the present invention include GAL1, GAL7, ADH2, PGK, GAPDH, etc. (Kingsman, S.M. et al., Biotechnology & Genetic Engineering Reviews, Vol 3, 377-416, (1985); Russell, D.W. et al., The Journal of Biological Chemistry, Vol 258, No.4, 2674-2682 (1983)); and AOX1 (Digam, et al., Dev. Ind. Micro. Biol, 29, 59-65, (MS8)). Use of the inducible promoter such as the GAL1, GAL7 or ADH2 promoter may be preferred as it enables expression to be controlled. Expression of the GAL1 and GAL7 promoters is induced by galactose.

An appropriate expression vector may be obtained by cloning a DNA sequence according to the present invention into an expression vector. An example of a complete expression vector, containing the GAL1 promoter, is pWYG5-TET15 which contains the whole synthesised DNA encoding fragment C (see Figure 12).

In a further aspect of the invention there is provided a yeast organism transformed with an expression vector according to the invention.

Examples of suitable host cells for use in the above-described method are yeast cells such as Saccharomyces, Pichia, Kluyveromyces or Hansenula and in particular the following species; Saccharomyces cerevisiae, Kluyveromyces lactis, Hansenula polymorpha, or Pichia pastoris.

A strain of yeast which can be used is Saccharomyces cerevisiae strain S150-2B.

The present invention provides a process for the preparation of fragment C of tetanus toxin which process comprises culturing a transformed yeast organism according to the invention and recovering the product fragment C thus expressed.

The process may be affected by:

(i) preparing the DNA of fragment C to contain codons of increased (G+C)-content by chemically synthesising the entire coding sequence

(ii) inserting the DNA into a suitable vector

(iii) transforming yeast cells

(iv) culturing a transformed host to express fragment C of tetanus toxin

(v) recovering the product fragment C thus expressed

Recombinant tetanus toxin fragment C may therefore be obtained thus facilitating its use as the basis for an alternative vaccine to formaldehyde treated tetanus toxoid and tetanus toxin fragment C as expressed in E.coli.

Step (iv) of the process of the invention comprises culturing yeast transformed by the expression vector of the present invention such as to cause expression of fragment C. Fragment C may then be isolated from the yeast cells by for example breaking the yeast cells with glass beads or when the material is secreted by isolation from the culture medium

The DNA sequence and corresponding amino acid sequence encoded by plasmid pWYG5-TET15 mentionned below is shown in Figure 2 and in SEQ ID NO:2. The symbol ,,, is shown under the translational stop codon. The nucleotide changes made in the synhestised gene are shown below the original C.tetani DNA sequence.

The fragment C that is expressed is recovered, in step (v) of the present process, from the yeast cells by similar protocols by standard purification procedures. (Makoff, A.J., et al., Bio/Technology, 7, 1043-1046, (1989)).

The fragment C may be isolated to the desired degree of purity. Some minor yeast contaminants may also be present. Generally the degree of purity is at least 80%, preferably at least 90% and more preferably at least 95%.

A vaccine for conferring immunity to tetanus may be formed by formulating tetanus toxin fragment C prepared according to the invention with a pharmaceutically acceptable carrier or diluent. The vaccine may include other antigens to provide a multi-valent vaccine. Typically carriers and diluents are sterile, pyrogen-free liquid media suitable as vehicles for introducing a polypeptide into a patient. Isotonic saline solution may be employed.

The vaccine may also comprise an adjuvant for stimulating the immune response and thereby enhancing the effect of the vaccine. A convenient adjuvant is aluminium hydroxide. Conveniently the vaccines are formulated to contain a final concentration of fragment C or its derivative of from 0.2 to 200 μg/ml, preferably 5 to 50 μg/ml, most preferably about 30μg/ml. After formulation the vaccine may be incorporated into a sterile container which is then sealed and stored at a low temperature, for example 4°C, or it may be freeze-dried.

The vaccine may be administered by any conventional method for the administration of vaccines such as parenteral (e.g. subcutaneous or intramuscular) injection. The treatment may consist of a single dose of vaccine or a plurality of doses over a period of time. It is recommended that each dose is 0.1 to 2ml preferably 0.2 to 1ml, most preferably about 0.5ml of vaccine.

The inventors have surprisingly found that it is possible to secrete fragment C into the culture medium using an appropriate secretion signal such as the alpha factor leader peptide. The protein was found to be secreted to a level of 5-10mg/l into the medium and was present in two forms in roughly equal amounts: a high molecular mass hyper-glycosylated protein (75-200kDa), and a core-glycosylated protein (65kDa). This glycosylated protein was found to be substantially inactive in vaccinating mice against tetanus toxin. However, if the glycosylated protein is de-glycosylated it becomes as active as the intracellular fragment C in immunising against tetanus.

As it should be possible to secrete fragment C to levels in excess of 100mg/l in high-density fermentations the de-glycosylated secreted product may provide a feasible production alternative to the intracellular protein production.

The invention will be described in more detail hereinafter with reference to the accompanying drawings in which:

Figure 1 shows the location of elements responsible for the production of incomplete transcripts identified in the four variants of fragment C DNA having different amounts of synthesised DNA. Coding regions for fragment C are boxed; regions that were chemically synthesised with codons optimal for translation in E.coli are hatched. The four versions of the gene, TET2, TET7, TET11 and TET15, had 12%, 50%, 73% and 99% synthetic DNA, respectively. The approximate positions of yeast polyadenylation sites found in the native sequence, estimated from the sizes of short transcripts in Northern blots, are indicated by arrows. (The 5' synthesised region in TET2 extends 160nt into the gene, and the first terminator is at 560 ± 50nt).

Figure 2 shows the sequence of C. tetani DNA encoding fragment C (top line), the nucleotide changes made in the fully synthetised version of fragment C (middle line) and the amino acid sequence (third line) (SEQ ID NOS:1 and 2).

Figure 3 shows the construction of the yeast expression vector pWYG 7. foreign genes are inserted between the Bam HI and Bcl I sites.

Figure 4 shows the nucleotide sequence of the promoter region of GAL7 (SEQ ID NO:3). The synthetised promoter corresponds to the XhoI to BamHi fragment. Regions downstream of BamHI are present in native GAL7 including the RNA start site (↓) and the initiating ATG (underlinded). The two basepairs which were altered to give a BamHI site are underlined.

Figure 5 shows the construction of yeast expression vector pWYG5.

Figure 6 shows the map of pTETtac2.

Figure 7 shows the E. coli vector for expression of tetanus toxin fragment C (pTETtac2) with progressively more synthetised DNA containing optimal codons. Only the region between the EcoRI and AvaI site is shows, the full map of pTETtac2 being given in Fig.6. The fragment C coding regions are boxed and synthetised regions are hatched.

Figure 8 shows the construction of pTETtac16. The oligonucleotides inserted into pTETtac7 to obtain pTETtac14 are shown in SEQ ID NOS:4 and 5.

Figure 9 shows the construction of pWYG7-TET2. The oligonucleotides inserted into pTETtac2 to obtain pTETtac2Y are shown in SEQ ID NOS:6 and 7.

Figure 10 shows a Western blot analysis of proteins from induced cells containing no plasmid, pWYG7-TET2, pWYG5-TET7, pWYG5-TET11 or pWYG5-TET15 (tracks 1 to 5, respectively). Track 6 was loaded with Met-fragment C produced in E.coli. The proteins (50μg) were separated in a 9% SDS-polyacrylamide gel. blotted onto nitrocellulose, and probed with a rabbit anti-fragment C serum as first antibody. Track 3 contains a very faint doublet at about 30kDa which is not visible in the reproduction.

Figure 11 shows a Northern blot of RNA extracted from induced cells transformed with pWYG7-TET2, pWYG5-TET7, pWYG5-TET11 and pWYG5-TET15 (tracks 1 to 4 respectively). The position of stained RNA size markers (size in kb) is indicated. The blot was probed with $^{32}P$-labelled 1.4kb BglII-BamHI fragment of pTETtac2.

Figure 12 shows the map of pWYG5-TET15

Figure 13 shows the nucleotide sequence of the synthetic DNA fragments carrying the α-factor prepro region used in pWYG69-TET2 and pWYG59-TET15 (SEQ ID NO : 8)

Figure 14 shows a Western blot of secreted yeast fragment C. Lane 1, pWYG59-TET15 culture supernatant treated with endoglycosidase H. Lanes 2 and 3, untreated pWYG59-TET15 culture supernatant. Lane 4, pWYGG-TET2 culture supernatant. Lane 5, pWYG69-TET2 culture supernatant treated with endoglycosidase H. Lane 6, culture supernatant from untransformed cells. Lane 7, culture supernatant from untransformed cells after endoglycosidase H treatment. Lane 8, molecular weight markers. Lane 9, fragment C produced in E. coli.

Figure 15 shows the construction of pPIC3-TET15. The oligonucleotides inserted in pAO804 to obtain pPIC1 are shown in SEQ ID NOS: 9 and 10.

Figure 16 shows fragment C production in different pPIC3-TET15 transformants. Parc a) shows proteins from total cell extracts separated on a Coomassie blue stained SDS-polyacrylamide gel. Lanes 1-11 are loaded with extracts from clones 885C, 887C, 8811C, 8812D, 881D, 882E, 885E, 8811E, 8810F, 883H respectively. Lane 12, extract from fragment C expressing E. coli. Lane 13, molecular weight markers (phosphorylase b, 97,400; bovine serum albumin, 68,000; ovalbumin, 43,000; chymotrypsinogen, 25,700; lactoglobulin, 18,400). Lane 14, insoluble fraction from 881F. Lane 15, total extract from 881F. Lane 16, soluble fraction from 881F. Part b) shows a Western blot of these samples. Lanes 1-9, as in part a). Lane 10, extract from 889F. Lane 11, extract from 8810F. Lane 12, extract from 883H. Lane 13, extract from untransformed cells. Lane 14, molecular weight markers.

Figure 17 shows a Coomassie blue stained SDS polyacrylamide gel showing fragment C production in a high cell density fermentation of clone 881F. Lane 1, molecular weight markers (ß-galactosidase, 116,000; phosphorylase b, 97,400; bovine serum albumin, 68,000; ovalbumin, 43,000; carbonic anhydrase, 29,000). Lane 2, untransformed cell extract. Lane 3, 881F extract from an induced shake-flask culture. Lanes 4-14, extracts from cells taken from the fermenter at the following time intervals with respect to the beginning of induction, -15,0,2,4,6,8,24,28,30,32,52 hours.

The following Examples illustrate the present invention and are not intended to limit the invention in any way.

## EXAMPLE 1

1. Construction of yeast expression vector pWYG7

The vector pWYG7, (Beesley,K.M., et al., Bio/Technology, 8, 644-649 (1990)), constructed at Wellcome, was used for the expression of C fragment. The construction of pWYG7 is outlined in Figure 3. It is derived from the 2u vector pJDB219 (Beggs, J.D., Nature, 275, 104-109, (1978)) modified to contain a kanamycin-resistance marker ($kan^r$) and the yeast galactose-regulated GAL7 promoter. First the $kan^r$ marker (HincII fragment from pUC4K; Vieira, J. and Messing, J., Gene, 19, 259, (1982)) was ligated into the unique SmaI site of pJDB219 to give the $kan^r$ $tet^r$ vector pJDB219K. Secondly, a synthetic GAL7 promoter fragment (XhoI-BamHI fragment, sequence shown in Figure 4 and in SEQ ID NO:3) was cloned between the unique SalI and BamHI sites of pJDB219K. The resulting vector, pWYG7, has the GAL7 promoter with unique BamHI and BclI sites upstream of the yeast the 2u plasmid FLP gene transcriptional terminator (Sutton, A., and Broach, J.R., Mol.Cell.Biol, 5, 2770-2780 (1985)). Foreign genes to be expressed form pWYG7 are inserted between the BamHI and BclI sites. The design of the GAL7 promoter fragment is discussed below.

The smallest fragment of DNA upstream of the GAL7 gene which exhibits full promoter activity has been defined by deletion mapping (Tajima, M., et al., Yeast, 1, 67-77, (1985)). Based on this information a 260 bp GAL7 promoter fragment was synthetised (Figure 4 for sequence). The 260bp promoter was synthetised as four overlapping oligonucleotides using a Pharmacia Gene Assembler (protocol supplied by Pharmacia). These oligonucleotides were phosphorylated and annealed using standard techniques, then ligated into XhoI-BamHI cut pIC-20H (Marsh, J.C., Gene 32, 481-485, (1984)). Positive clones were identified and their DNA sequenced using the double-stranded DNA sequencing method with universal and reverse sequencing primers (Hong, G.F., Biosc, Reports 2, 907, (1982)). The sequence of the GAL7 inserts was confirmed, and then the XhoI-BamHI GAL7 insert was excised and cloned into pJDB219K as described above.

The design of the GAL7 promoter fragment in pWYG7 is such that the natural GAL7 DNA sequence has been slightly modified (2bp changed) in order to make the BamHI cloning site upstream of the GAL7 mRNA start sites. The foreign gene to be expressed is then linked with synthetic DNA to the BamHI site, such that the GAL7 mRNA start sites are introduced, along with the GAL7 upstream untranslated sequences. Thus the first non-yeast DNA downstream of the promoter is the initiating ATG codon of the foreign gene, and the transcript produced will have a yeast GAL7 leader rather than a foreign leader which could reduce efficiency of translations.

## EXAMPLE 2

Construction of yeast expression vector pWYG5

The vector pWYG5 is the same basic plasmid as pWYG7 but has the GAL1 promoter from pBM150 (Johnston, M. and Davis, R.W. Mol. Cell. Biol 4, 1440-1448 (1984)) in place of the GAL7 promoter. The 0.7kb EcoRI-BamHI fragment from pBM150, containing the divergent GAL and GAL10 promoters, was first sub-cloned be-

tween the EcoRI and BamHI sites of pIC-20H (Marsh et al., J.C., Gene 32, 481-485, (1984)) to give pIC-GAL, then the 0.7kb XhoI-BamHI promoter fragment from pIC-GAL was isolated and placed between the SalI and BamHI sites of pJDB219K to give pWYG5 (the construction is outlined in Figure 5).

The GAL1 promoter from pBM150 has a BamHI linker placed downstream of the RNA initiation sites and therefore pWYG5 is used differently from pWYG7. Foreign genes must be adapted to have a BamHI or BamHI-compatible (i.e. BglII or BclI) site immediately upstream of the initiation codon. In order to conform with the consensus found in highly expressed yeast genes, the sequence upstream of the ATG should be rich in A residues and particularly have A at -3. As with pWYG7, the foreign genes are inserted between the BamHI and BclI sites of pWYG5.

## EXAMPLE 3

Construction of E.coli expression vectors for tetanus toxin fragment C, including synthesised versions of the gene, and intermediate vectors for yeast expression.

Expression cassettes of fragment C DNA for transfer to the yeast vectors pWYG5 and pWYG7 were isolated from the E.coli expression vector pTETtac2 and its derivatives (Makoff et al., 1989; U.K. patent application No. 89141220.0). pTETtac2 is a tac promoter vector containing DNA coding for Met-fragment C (Figure 6 for plasmid map); the first 161bp and last 42bp of the natural C.tetani DNA have been replaced by synthesised DNA which was altered to optimise codon usage for E.coli and to provide useful restriction sites. (All synthetic DNA was chemically synthesised as oligonucleotides of length 50-160, on a Pharmacia Gene Assembler, which were phosphorylated, annealed and assembled into the relevant plasmids). Expression vectors based on pTETtac2 were then constructed where progressively more of the C.tetani DNA, starting from the 5' end, was replaced by synthesised DNA whose codon usage was optimised for E.coli. The first vector pTETtac7, was constructed via the intermediate plasmid pTETtac6, shown in Figure 7; pTETtac7 contains an approximately 45% synthesised gene. This involved cloning two oligonucleotides between the BanI and MaeII sites of pTETtac2 in order to produce the two unique sites NcoI and AflII in pTETtac6. Eight more oligonucleotides were then cloned between the two sites to generate pTETtac11, which contained a 75% synthesised gene.

A version of pTETtac2 containing the 99% synthesised gene (pTETtac15) for fragment C was actually first designed specifically as an intermediate vector (pTETtac16) for transfer of the expression cassette to the yeast vector pWYG5. The nucleotide sequence of the synthetised gene is compared to the original C.tetani gene in Figure 2 from this sequence and the restriction maps in Figure 7 the sequence of each version of the gene can be derived. The overall scheme for the construction of pTETtac16 is shown in Figure 8. First, pTETtac7 was modified by replacement of the DNA between the BglII and SalI sites with oligonucleotides which provided upstream sequences compatible with the yeast vector pWYG5 (sequence of oligonucleotides in Figure 8 and in SEQ ID NOS: 4 and 5). Secondly, the remaining 400bp of the DNA encoding fragment C was synthesised as four oligonucleotides of length 140 to 160. These were phosphorylated, annealed and cloned between the ClaI and BamHI sites of pIC-20H. Recombinant plasmids containing the 400bp insert were identified and further checked by sub-cloning into M13 and sequencing (Sanger. F., et al., Proc.Nat.Acad.Sci., 74, 5463-5467, (1977)). A plasmid with an insert of the correct sequence, designated pIC-TET, was used as a source of the 400bp ClaI-BamHI fragment to ligate to the 4199bp AflII-BamHI fragment of pTETtac14 and the 325bp AflII-ClaI fragment of pTETtac11 in order to create pTETtac16. pTETtac16 then has the fully synthesised gene for fragment C with codons optimised for E.coli and considerably more (GC)-rich DNA than the C.tetani DNA, preceded by an upstream region suitable for expression in pWYG5.

## EXAMPLE 4

Construction of yeast intracellular expression vectors for fragment C.

Four vectors were constructed, one based on pWYG7 and three on pWYG5. The pWYG7 vector, pWYG7-TET2, contained the largely unaltered form of the natural C.tetani gene from pTETtac2. The remaining vectors, pWYG5-TET7, pWYG5-TET11 and pWYG5-TET15 were all based on pWYG5 and contained the genes with progressively more synthesised DNA, from the plasmids pTETtac7, pTETtac11 and pTETtac16, respectively.

(i) pWYG7-TET2

The DNA between the BglII and SalI sites of pTETtac2 was replaced by two oligonucleotides to give pTETtac2Y in order to provide the GAL7 upstream sequences required for expression in pWYG7 (Figure 9 for construction and sequences; sequences also shown in SEQ ID NOS:6 and 7). The oligonucleotides also placed an NcoI (GGATGG) site at the initiating ATG, altering the second codon from Lys to Val. The 1.4kb BglII-BamHI

fragment from pTETtac2Y was isolated and ligated with pWYG7 (dam-DNA) which had been digested with the BamHI and bclI and then with calf intestinal alkaline phosphatase. Recombinant plasmids with inserts of the correct orientation were designated pWYG7-TET2.

Western blot analysis of protein extracts from induced cells containing pWYG7-TET2 gave no detectable product reacting with the antibody (track 2, Figure 10). An ELISA quantitation gave an exceeding low, but positive, figure of less than $10^{-3}$ of soluble protein. Since the gene for fragment C was found to be efficiently expressed in a number of other host cells, the plasmid and transformants were rechecked and expression reanalysed extensively.

A gene encoding an unmodified fragment C was next tested.

(ii) pWYG5-TET7 and pWYG5-TET11

These plasmids were made by transferring the 1.4kb BglII-BamHI fragments pTETtac7 and pTETtac11 into pWYG5, between the BamHI to BclI sites. The transcripts produced by pWYG5-TET7 and pWYG5-TET11 in yeast may be translated sub-optimally since the upstream regions between the BglII site and the initiation codon are designed for E. coli expression, and do not conform to the consensus for highly expressed yeast genes.

Western blot analysis of the products from induced cells containing the plasmid pWYG5-TET7 showed the presence of two faint bands at approximately 29kDa and 30kDa (track 3, Figure 10 - too faint to see in reproduction), but no full length fragment C (approximately 50kDa). This result provided the clue that incomplete transcripts were being produced, therefore the fragment C-specific mRNA from pWYG7-TET2 and pWYG7-TET7 was analysed. The Northern blot (Figure 11) showed that instead of a full-length transcript (expected size approximately 1655 nucleotides), pWYG7-TET2 gave rise to a major band of approximately 700 nt and a minor band of 600 nt and pWYG7-TET7 to two bands of approximately 900 and 1100 nt. Since these RNAs all hybridised to a probe from the 5' end of the gene (BglII to NcoI fragment from pTETtac7), incomplete transcripts were being produced within the gene for fragment C. The fact that the transcripts from pWYG-TET7 are larger suggests that the original C.tetani DNA contained sequences which do not allow the production of complete mRNA transcripts, and that these were destroyed as the DNA was synthesised. This idea is reinforced by the fact that the approximate position of the elements involved with the production of an incomplete mRNA transcript in pWYG7-TET2 is within the region which was modified by synthesis in pWYG5-TET7.

Induced cells containing pWYG5-TET11 were shown to produce fragment C of the correct size on Western blots (Figure 10), at a concentration of approximately0.5% of cell protein. The product was soluble in that it remained in the supernatant after centrifugation at 10,000g for 15 min.

An analysis of RNA from pWYG5-TET11 (Figure 11) showed that there were two major transcripts of less than full length (1200 and 1400 nt), and only a minor amount of the full length transcript (approximately 1700 nt). Thus efficient expression was still being prevented by the remaining 400bp of C.tetani DNA in the pWYG5-TET11 plasmid.

(iii) pWYG5-TET15

The 1.4kb BglII-BamHI fragment of pTETtac16 was isolated and cloned between the BamHI and BclI sites of pWYG5 as described above. Plasmids with inserts of the correct orientation were designated pWYG5-TET15 (Figure 12).

Cells containing this plasmid produced greater amounts of fragment C than before, to a level of 2.5% of cell protein (ELISA quantitation and see Figure 10). Analysis of the RNA indicated that for the first time most of the fragment C-specific RNA was full length (Figure 11). Thus it must be concluded from the RNA analysis that the C.tetani DNA encoding fragment C contains at least six elements which are fully or partially responsible for the production of incomplete transcripts in yeast. The positions of the elements are shown in Figure 1. With the present state of knowledge about transcription of mRNA in yeast few if any of these could be predicted from the sequence of the DNA, and they could be removed by re-synthesising the DNA to have a higher (G + C)-content. Alternatively, the elements could be accurately delineated by mapping of the 3' ends of the truncated transcripts described above, and only those regions identified as being responsible for the production of incomplete transcripts being resynthesised.

**EXAMPLE 5**

Construction of yeast secretion vectors for fragment C

Two vectors were constructed for the secretion of fragment C, pWYG9-TET2 and pWYG59-TET15. These both contained DNA encoding the prepro leader peptide from the yeast mating pheromone, alpha-factor (Kur- jan, J. and Herskowitz, I., Cell 30, 933-948, (1982)).

(i) pWYG9-TET2

This vector is similar to pWYG7-TET2 but contains the coding region for the alpha-factor leader peptide between the BamHI site of the GAL7 promoter and the NcoI site at the initiating ATG codon of fragment C. The synthetic DNA fragment contains altered codons, in order to generate a XhoI restriction site to facilitate cloning, giving a conservative amino acid change (Asp to Glu) immediately upstream of the KEX2 cleavage site. GAL7 upstream sequences required for expression in pWYG7 are also included (Figure 13).

In Western blots of culture supernatants from cells transformed with pWYG9-TET2 a broad smear of reactive material of heterogenous molecular weight (75-200kD) was observed. When de-glycosylated with endoglycosidase H the molecular weight of this was substantially reduced to a major species of approximately 26kD (Figure 14). This result gave further support for the notion that the wild-type C.tetani fragment C gene contains sequences fortuitously recognised as being responsible for the production of incomplete mRNA transcripts. The size of this band is consistent with it being a run-off translation product of the major transcript characterised by Northern analysis (Example 4).

(ii) pWYG59-TET15

This vector is similar to pWYG5-TET15 but contains the alpha-factor leader peptide coding region between the BamHI site of the GAL1 promoter and the SalI site near the 5' end of the fragment C gene. The synthetic DNA fragment also contains the same NcoI site found at the initiator ATG of pWYG7-TET2 (see Figure 13).

Two forms of fragment C were found to be secreted into the medium by cells containing pWYG59-TET15. A diffuse band of high molecular weight material was detected similar to that seen with pWYG9-TET2. In addition, a major band of about 65kD was detected (Figure 14). A ladder of at least four other less intense bands of lower molecular weight was also visible. All of these species were reduced to approximately 50kD, the size expected for correctly processed full length fragment C, when treated with Endo H suggesting that the differences between them are due to differences in N-linked glycosylation. Fragment C contains seven potential sites for the addition of asparagine-linked carbohydrate and our data suggests that at least five of these are actually being used during alpha-factor signal directed secretion.

Secretion of full length fragment C by yeast cells containing the resynthesised TET15 gene was found to be efficient. The total amount of fragment C secreted to the medium by unoptimised shake flask cultures was estimated to be about 7μg/ml and none was detected in intracellular extracts from these cultures.

## EXAMPLE 6

Construction of Pichia pastoris intracellular expression vectors for fragment C.

The vector pP1C3-TET15, which is derived from pA0804, was used for intracellular expression of fragment C in Pichia (Diagan, et al., Dev.Ind. Microbiol., 29, 59-65, (1988); Sreekrishna et al., Biochemistry, 28, 4117-4125 (1989)). This vector uses the promoter from the AOX1 gene to drive expression and can be integrated into the host chromosomal AOX1 locus.

To facilitate insertion of the fragment C gene the synthetic adapter oligonucleotides shown in Figure 15 and in SEQ ID NOS:9 and 10 were cloned between the AsuII and EcoRI sites of pA0804, to give pPIC1. A derivative of this plasmid, pPIC2, which lacks the EcoRI site was then constructed. This was done by digesting with EcoRI followed by filling in of the protruding single stranded ends with the Klenow fragment of DNA polymerase I and the blunt ends were then ligated together. The 1.4kb BglII-NheI fragment from pTETtac16 containing the fragment C gene was then inserted between the BamHI and SpeI sites of pPIC2 to give pP1C3-TET15 as shown in Figure 15.

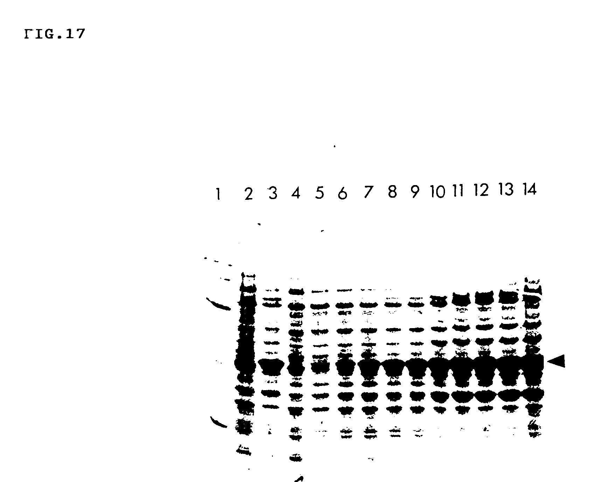

Fragment C production in shake flasks, by several pPIC3-TET15 transformants that grew slowly on methanol, was examined. Figure 16 shows SDS-PAGE and Western blotting analysis of cell lysates. Expression levels were estimated by densitometric scanning of Coomassie blue stained gels and by ELISA and these varied between different transformants from 0.3% of total cell protein to about 11%. Even at the highest level of expression the product was soluble. The highest expressing strain, 881F, was used in high cell density inductions in a fermenter. Cells were grown to a density of 90g/l (dry weight) before induction. A time course for the induction is shown in Figure 17. Production of fragment C began rapidly upon induction, rose to a level of about 20-28% of total cell protein after 24 hrs and remained at this level up to 52 hrs after induction. The final level of fragment C in the fermenter was estimated to be about 11g/l and again the product was soluble.

## EXAMPLE 7

Transformation of yeast with fragment C expression vectors

The vectors were introduced into the Saccharomyces cerevisae strain S150-2B (a leu2 his3 ura3 trpl; (McCleod, M., et al., Cold Spring Harbor Symp., Quant., Biol., 49, 779-787, (1984)) using the lithium transformation procedure of Ito et al. J. Bact., 153 163-168, (1983). Transformed yeast cells were incubated in YPD broth (Sherman, F., et al., Methods in Yeast Genetics, Cold Spring Harbour, N.Y., 1983) at 30°C overnight prior to plating out on selective medium (YPD plus 500ug/ml G418). This allows expression of G418-resistance and increases transformation frequency. Colonies that came up as G418$^r$ were tested on minimal medium lacking leucine (YNB, Difco + glucose + histidine + uracil + tryptophan, Sherman et al., 1983) to test for the Leu$^+$ phenotype also conferred by the plasmids. Positive transformants (G418$^r$ Leu$^+$) were used for expression analysis.

The vector pPIC3-TET15 was introduced into Pichia pastoris strain GS115 using the sphaeroplast transformation procedure described by Cregg et al, (1985). (Cregg et al., Molecular and Cellular Biology, 5, 3376-3385 (1985)) To direct integration into the host chromosomal AOX1 locus the vector was digested with Bgl II and then mixed, in the presence of calcium ions and polyethylene glycol, with sphaeroplasts generated by enzymatic digestion of the cell walls with zymolyase. Transformed sphaeroplasts were regenerated in osmotically buffered agarose containing YNB, glucose (2%), sorbitol (1%) biotin (400 µg/l) and His-assay medium (Difco). Transformed cells were tested for growth on methanol since those disrupted at AOX1 by insertion of the vector should grow slowly on methanol.

## EXAMPLE 8

Galactose induction and preparation of cell lysates

Transformants were grown to the mid-logarithmic stage ($10^7$ cells/ml) in YP broth containing 2% raffinose and 500 µg/ml G418 at 30°C in an orbital shaker. An aliquot of 40% galactose was then added to a final concentration of 2% and the culture was incubated for a further 24h. The cells were harvested by low speed centrifugation, washed once in distilled water, and resuspended in ice-cold break buffer (20mM sodium phosphate pH7.0, 0.1% triton X-100, 4mM phenylmethyl sulphonyl fluoride, 4mM EGTA, and 2µg/ml each of pepstatin, antipain, leupeptin and chymostatin; 5ml for cells from a 250ml culture). Acid-washed glass beads (0.45mm) were added and the cells were broken by vigorous vortexing. In order to remove insoluble proteins, the crude cell lysate could be cleared by centrifugation for 15min at 10,000g. The protein concentration of the extracts was determined using the BioRad protein assay (BioRad, according to manufacturer's instructions) and the material was stored at -70°.

## EXAMPLE 9

Methanol Induction of Pichia cultures.

Transformants were grown at 30°C overnight to saturation in liquid minimal medium (YNB containing biotin, 400µg/l, and glycerol, 2% v/v). 1ml aliquots of these cultures were used to inoculate shake flasks containing 10 mls of the same medium plus 1% casamino acids. After 6-8 hrs incubation at 30°C the cells were harvested by centrifugation and resuspended in YNB (Difco) containing biotin (400µg/l) casamino acids (1%), and methanol (0.5% v/v). After further incubation for 2-6 days the cells were harvested and lysates prepared as described for Saccharomyces (see Example 8).

Production of fragment C by high cell density Pichia pastoris cultures was carried out using a 21 Braun fermenter equipped with monitors and controls for pH, dissolved $O_2$, stirring speed, temperature and air flow. A 10ml YNB + biotin + 2% glycerol overnight culture was used to inoculate the fermenter containing 1 litre of 5X basal salts (phosphoric acid, 42mls/l; calcium sulphate.2$H_2O$. 1.8g/l; potassium sulphate, 28.6g/l; magnesium sulphate.7$H_2O$, 23.4g/l; potassium hydroxide, 6.5 g/l) with 4mls of $PTM_1$ salts (cupric sulphate.5$H_2O$, 6g/l; potassium iodide, 0.08g/l; manganese sulphate.$H_2O$, 3g/l; sodium molybdate, 0.2g/l; boric acid, 0.02g/l; cobalt chloride, 0.5g/l; zinc chloride, 20g/l; ferrous sulphate.7$H_2O$, 65g/l; biotin, 0.2g/l; sulphuric acid 5mls/l) and 5% (v/v) glycerol at 30°C. Dissolved $O_2$ was maintained above 20% by adjusting aeration and agitation, and the pH was maintained at pH5.0 by the addition of 50% (v/v) ammonium hydroxide. Growth was continued until the glycerol was exhausted (24-30 hrs). A limited glycerol feed (containing 50% w/v glycerol and 12ml/l $PTM_1$ salts) was then initiated at 12 mls/hr for 17-21 hrs. After this period the culture was induced by replacing the glycerol feed with a methanol feed (100% methanol plus 12ml/l $PTM_1$ salts) at 1ml/hr. for 2 hrs. Then the methanol feed rate was gradually increased over a period of 6 hours to 6mls/hr and the fermentation was continued using these conditions for a further 46-92 hrs.

## EXAMPLE 10

Concentration of culture supernatants and glycoprotein analysis

Cells were induced and then harvested by centrifugation as described in Example 8. Culture supernatants were concentrated by ultrafiltration using Centricon 30 microconcentrators (Amicon), centrifuging at 4,000g for 45'. Supernatants from larger scale cultures were concentrated by ultrafiltration with Amicon PM30 membranes using a stirred cell. N-linked oligosaccharides were removed by digestion of concentrated supernatants with Endoglycosidase H (Endo H, Boehringer Mannheim). Aliquots (25µl) were taken and 5µl of digestion buffer added (0.2M $NaH_2PO_4$, 10mM B-mercaptoethanol, 1% SDS). After boiling for 5 minutes samples were cooled on ice and protease inhibitors added to the same final concentrations as given above (Example 8). Endo H (9mU) was added and the samples were incubated for 18hrs at 37°C before analysis by SDS-PAGE (Example 11).

## EXAMPLE 11

SDS-polyacrylamide gel analysis of proteins

Soluble or total protein extracts from induced yeast cells were separated by electrophoresis in SDS-polyacrylamide gels (Laemmli, UK., Nature, 227, 680-685, (1970)). The proteins in the gel could be visualised by staining with Coomassie Brilliant Blue R. Alternatively the proteins were transferred to a nitrocellulose filter and reacted with rabbit antiserum to fragment C (isolated from C.tetani) and then goat anti-rabbit IgG conjugated to horse-radish peroxidase followed by colour development with hydrogen peroxide and 4-chloronaphthol (BioRad). In this way the expressed fragment C could be specifically detected.

## EXAMPLE 12

Immunoassay quantitation of fragment C

A two antibody sandwich enzyme-linked immunosorbent assay (ELISA) for fragment C was developed. Purified pepsinised antibody prepared from horses hyperimmunised with tetanus toxoid (Hughes et al., J. Appl.Bact. 38, 1974, 603-622) was used to coat flexible polyvinylchloride microtitre plates (Dynatech, Billinghurst, GB). Coating was performed overnight at 40°C in 50mM sodium carbonate pH 9.5 (12.5 µg pepsinised antibody per ml, 100µl per well). Plates were washed three times with phosphate buffered saline pH 7.2, 0.12 (w/v) Tween (Trade Mark) 20 (PBS-Tween).

Non-specific binding was reduced by incubation of plates with PBS-Tween containing 12% (wt/vol) BSA or 5% (wt/vol) non-fat dry milk. Plates were then incubated sequentially with antigen, second antibody and anti-rat IgG peroxidase conjugate (Kinkegaard and Perry, Maryland, US) for 1 hr at 37°C in each case. The second antibody was a rat monoclonal (TT09) which binds with high affinity to fragment C (Sheppard et al., Infec. Immun. 43, 1984, 710-714) and it was used at a concentration of 2µg/ml.

Anti-rat peroxidase conjugate was used at a dilution of 1:3000. Each reagent was diluted into PBS-Tween containing 5% non-fat dry milk prior to addition to plates. Plates were washed three times with PBS-Tween after each incubation. Bound enzyme complex was assayed using tetramethylbenzidine (TMB) as the chromogenic substrate. lmg TMB tablets (Wellcome Diagnostics) were dissolved in 10ml of 0.0625M trisodium citrate containing 0.01% hydrogen peroxide. 100µl of reagent was added to each well and the reaction terminated by addition of 100µl 2M $H_2SO_4$ after incubation for 10-15 mins at room temperature. Plates were read using a Titertek multiscan plate reader (MCC/340) at 450nm.

For quantitation, fragment C prepared from C.tetani (Fairweather, N., et al., J.Bact., 165, 21-27, (1986)) was used as a standard. Several dilutions of this fragment C gave a narrow range titration curve with a linear portion between about 1µg/ml and 10µg/ml. Titration curves for the recombinant fragment C were comparable, with similar slope and range to the standard curve. The titre of unknown samples were determined either by comparing the midpoints of titration curves or more routinely by reading directly from the linear portion of the standard curve.

Protein was determined using the BCA assay reagent (Pierce) with bovine serum albumin as protein standard.

## EXAMPLE 13

Preparation and analysis of RNA from yeast cells

In order to prepare RNA, transformed yeast cells were grown in YP + 2% raffinose + 500µg/ml G418 to a density of approximately $5x10^5$ cells/ml, then induced for 24 hr after addition of 2% galactose. Total RNA was then prepared from the cells and analysed by Northern blotting as described previously (Romanos, M.A., and Boyd, A., Nucl. Acids Res., 16, 7333-7350, (1988)). The Northern blots were probed with fragments of pTETtac2 DNA, and its derivatives, labelled by the random priming method (Feinberg, A., and Vogelstein, B., Anal. Biochem, 132, 6-13, (1983)).

## EXAMPLE 14

Immunisation of mice

Fragment C was purified from lysates of induced cells harbouring pWYG5-TET15 by affinity chromatography using TT08 monoclonal antibody (Sheppard, A.J., et al., Infect. Immun., 43 710-714 (1984)) linked to cyanogen bromide - activated sepharose 4B. Fragment C was eluted with 0.1M sodium citrate pH3.0 and neutralised by addition of one volume of 0.1M sodium phosphate pH7.0. Secreted fragment C was prepared by concentration of supernatants from induced cultures harbouring pWYG59-TET15 without further purification.

Vaccines containing fragment C were prepared with 10% Alhydrogel (trade mark) and serial dilutions were prepared. Balb/C mice were injected with 0.5ml then challenged four weeks later with 100 $LD_{50}$ of tetanus toxin. Survivors were counted after a further four days. The results, summarised in the table below, show that yeast intracellular fragment C has at least equal potency to E.coli produced material, whereas the secreted fragment C is inactive.

| | Vaccine | Concentration of fragment C | Survivors |
|---|---|---|---|
| 1. | Yeast intracellular fragment C | 50 µg/ml | 5 |
| | | 12.5 µg/ml | 5 |
| | | 3.125 µg/ml | 5 |
| | | 0.78 µg/ml | 4 |
| 2. | Yeast secreted fragment C | 50 µg/ml | 0 |
| | | 12.5 µg/ml | 0 |
| | | 3.125 µg/ml | 0 |
| | | 0.78 | 0 |
| 3. | E.coli fragment C | 25 µg/ml | 3 |
| | | 6.25 µg/ml | 1 |
| | | 1.5 µg/ml | 0 |
| 4. | PBS (negative control) | 0 | 0 |

## EXAMPLE 15

Immunisation of mice (also using secreted de-glycosylated material)

Fragment C was purified from lysates of induced cells harbouring pWYG5-TET15 by affinity chromatography using TT08 monoclonal antibody (Sheppard, A.J., et al., Infect. Immun., 43, 710-714 (1984)) linked to cyanogen bromide - activated sepharose 4B. Fragment C was eluted with 0.1M sodium citrate pH3.0 and neutralised by addition of one volume of 0.1M sodium phosphate pH7.0. Secreted fragment C was prepared by concentration of supernatants from induced cultures harbouring pWYG59-TET15 without further purification. To de-glycosylate this material for immunisation experiments the concentrate was treated with Endo as described in Example 10 except without the addition of mercaptoethanol and SDS and without boiling the sample.

Vaccines containing fragment C were prepared with 10% Alhydrogel (trade mark) and serial dilutions were prepared. Balb/C mice were injected with 0.5ml then challenged four weeks later with 100 $LD_{50}$ of tetanus toxin. Survivors were counted after a further four days. The results, summarised in the table below, show that yeast intracellular fragment C was at least as effective as the E.coli derived material, which has previously been shown to be equivalent to C.tetani fragment C (Makoff et al., 1989a). In contrast, the secreted product was totally inactive. However, de-glycosylation of secreted fragement C with endo H rendered it as protective as the intracellular product, suggesting that important neutralising epitopes were masked by carbohydrate side-chains in the glycosylated form.

Protection of immunised mice against tetanus toxin challenge

| | Survivors after challenge[a] | | | | |
|---|---|---|---|---|---|
| Antigen (dose in μg) | 2 | 0.5 | 0.125 | 0.03 | 0.008 |
| E.coli fragment C | 4[b] | 2 | 0 | 0 | 0 |
| Yeast intracellular | 5 | 5 | 5 | 0 | 0 |
| Yeast secreted | 0 | 0 | 0 | 0 | 0 |
| Yeast secreted, de-glycosylated | 5 | 5 | 3 | 0 | 0 |
| Saline | 0 | | | | |

[a] Groups of five mice were challenged with 100$LD_{50}$ of tetanus toxin.

[b] Four mice in this group.

## SEQUENCE LISTING

| | |
|---|---|
| SEQ ID NO : | 1 |
| SEQUENCE TYPE : | nucleotide with corresponding protein |
| SEQUENCE LENGTH : | 1359 base pairs |
| STRANDEDNESS : | double |
| TOPOLOGY : | linear |
| MOLECULAR TYPE : | genomic DNA |
| ORIGINAL SOURCE : | |
| ORGANISM : | human |
| IMMEDIATE EXPERIMENTAL SOURCE : | |
| NAME OF CELL-LINE : | |
| FEATURES : | from 1 to 1359 |
| PROPERTIES : | human fragment C tetanus toxin gene |

```
ATG AAA AAT CTG GAT TGT TGG GTT GAT AAT GAA GAA GAT ATA GAT GTT     48
Met Lys Asn Leu Asp Cys Trp Val Asp Asn Glu Glu Asp Ile Asp Val
  1               5                  10                  15

ATA TTA AAA AAG AGT ACA ATT TTA AAT TTA GAT ATT AAT AAT GAT ATT     96
Ile Leu Lys Lys Ser Thr Ile Leu Asn Leu Asp Ile Asn Asn Asp Ile
             20                  25                  30

AAT ATC GAT ATA TCT GGG TTT AAT TCA TCT GTA ATA ACA TAT CCA GAT    144
Ile Ser Asp Ile Ser Gly Phe Asn Ser Ser Val Ile Thr Tyr Pro Asp
         35                  40                  45

GCT CAA TTG GTG CCC GGA ATA AAT GGC AAA GCA ATA CAT TTA GTA AAC    192
Ala Gln Leu Val Pro Gly Ile Asn Gly Lys Ala Ile His Leu Val Asn
     50                  55                  60

AAT GAA TCT TCT GAA GTT ATA GTG CAT AAA GCT ATG GAT ATT GAA TAT    240
Asn Glu Ser Ser Glu Val Ile Val His Lys Ala Met Asp Ile Glu Tyr
 65                  70                  75                  80

AAT GAT ATG TTT AAT AAT TTT ACC GTT AGC TTT TGG TTG AGG GTT CCT    288
Asn Asp Met Phe Asn Asn Phe Thr Val Ser Phe Trp Leu Arg Val Pro
                 85                  90                  95

AAA GTA TCT GCT AGT CAT TTA GAA CAA TAT GGC ACA AAT GAG TAT TCA    336
Lys Val Ser Ala Ser His Leu Glu Gln Tyr Gly Thr Asn Glu Tyr Ser
            100                 105                 110

ATA ATT AGC TCT ATG AAA AAA CAT AGT CTA TCA ATA GGA TCT GGT TGG    384
Ile Ile Ser Ser Met Lys Lys His Ser Leu Ser Ile Gly Ser Gly Trp
        115                 120                 125

AGT GTA TCA CTT AAA GGT AAT AAC TTA ATA TGG ACT TTA AAA GAT TCC    432
Ser Val Ser Leu Lys Gly Asn Asn Leu Ile Trp Thr Leu Lys Asp Ser
    130                 135                 140

GCG GGA GAA GTT AGA CAA ATA ACT TTT AGG GAT TTA CCT GAT AAA TTT    480
Ala Gly Glu Val Arg Gln Ile Thr Phe Arg Asp Leu Pro Asp Lys Phe
145                 150                 155                 160

AAT GCT TAT TTA GCA AAT AAA TGG GTT TTT ATA ACT ATT ACT AAT GAT    528
Asn Ala Tyr Leu Ala Asn Lys Trp Val Phe Ile Thr Ile Thr Asn Asp
                165                 170                 175

AGA TTA TCT TCT GCT AAT TTG TAT ATA AAT GGA GTA CTT ATG GGA AGT    576
Arg Leu Ser Ser Ala Asn Leu Tyr Ile Asn Gly Val Leu Met Gly Ser
            180                 185                 190

GCA GAA ATT ACT GGT TTA GGA GCT ATT AGA GAG GAT AAT AAT ATA ACA    624
Ala Glu Ile Thr Gly Leu Gly Ala Ile Arg Glu Asp Asn Asn Ile Thr
        195                 200                 205

TTA AAA CTA GAT AGA TGT AAT AAT AAT AAT CAA TAC GTT TCT ATT GAT    672
Leu Lys Leu Asp Arg Cys Asn Asn Asn Asn Gln Tyr Val Ser Ile Asp
    210                 215                 220

AAA TTT AGG ATA TTT TGC AAA GCA TTA AAT CCA AAA GAG ATT GAA AAA    720
Lys Phe Arg Ile Phe Cys Lys Ala Leu Asn Pro Lys Glu Ile Glu Lys
225                 230                 235                 240
```

```
TTA TAC ACA AGT TAT TTA TCT ATA ACC TTT TTA AGA GAC TTC TGG GGA    768
Leu Tyr Thr Ser Tyr Leu Ser Ile Thr Phe Leu Arg Asp Phe Trp Gly
                245                 250                 255

AAC CCT TTA CGA TAT GAT ACA GAA TAT TAT TTA ATA CCA GTA GCT TCT    816
Asn Pro Leu Arg Tyr Asp Thr Glu Tyr Tyr Leu Ile Pro Val Ala Ser
            260                 265                 270

AGT TCT AAA GAT GTT CAA TTG AAA AAT ATA ACA GAT TAT ATG TAT TTG    864
Ser Ser Lys Asp Val Gln Leu Lys Asn Ile Thr Asp Tyr Met Tyr Leu
        275                 280                 285

ACA AAT GCG CCA TCG TAT ACT AAC GGA AAA TTG AAT ATA TAT TAT AGA    912
Thr Asn Ala Pro Ser Tyr Thr Asn Gly Lys Leu Asn Ile Tyr Tyr Arg
    290                 295                 300

AGG TTA TAT AAT GGA CTA AAA TTT ATT ATA AAA AGA TAT ACA CCT AAT    960
Arg Leu Tyr Asn Gly Leu Lys Phe Ile Ile Lys Arg Tyr Thr Pro Asn
305                 310                 315                 320

AAT GAA ATA GAT TCT TTT GTT AAA TCA GGT GAT TTT ATT AAA TTA TAT   1008
Asn Glu Ile Asp Ser Phe Val Lys Ser Gly Asp Phe Ile Lys Leu Tyr
                325                 330                 335

GTA TCA TAT AAC AAT AAT GAG CAC ATT GTA GGT TAT CCG AAA GAT GGA   1056
Val Ser Tyr Asn Asn Asn Glu His Ile Val Gly Tyr Pro Lys Asp Gly
            340                 345                 350

AAT GCC TTT AAT AAT CTT GAT AGA ATT CTA AGA GTA GGT TAT AAT GCC   1104
Asn Ala Phe Asn Asn Leu Asp Arg Ile Leu Arg Val Gly Tyr Asn Ala
        355                 360                 365

CCA GGT ATC CCT CTT TAT AAA AAA ATG GAA GCA GTA AAA TTG CGT GAT   1152
Pro Gly Ile Pro Leu Tyr Lys Lys Met Glu Ala Val Lys Leu Arg Asp
    370                 375                 380

TTA AAA ACC TAT TCT GTA CAA CTT AAA TTA TAT GAT GAT AAA AAT GCA   1200
Leu Lys Thr Tyr Ser Val Gln Leu Lys Leu Tyr Asp Asp Lys Asn Ala
385                 390                 395                 400

TCT TTA GGA CTA GTA GGT ACC CAT AAT GGT CAA ATA GGC AAC GAT CCA   1248
Ser Leu Gly Leu Val Gly Thr His Asn Gly Gln Ile Gly Asn Asp Pro
                405                 410                 415

AAT AGG GAT ATA TTA ATT GCA AGC AAC TGG TAC TTT AAT CAT TTA AAA   1296
Asn Arg Asp Ile Leu Ile Ala Ser Asn Trp Tyr Phe Asn His Leu Lys
            420                 425                 430

GAT AAA ATT TTA GGA TGT GAT TGG TAC TTT GTA CCT ACA GAT GAA GGA   1344
Asp Lys Ile Leu Gly Cys Asp Trp Tyr Phe Val Pro Thr Asp Glu Gly
        435                 400                 445

TGG ACA AAT GAT TAA                                               1359
Trp Thr Asn Asp ***
    450
```

## SEQUENCE LISTING

| | |
|---|---|
| SEQ ID NO : | 2 |
| SEQUENCE TYPE : | nucleotide with corresponding protein |
| SEQUENCE LENGTH : | 1359 base pairs |
| STRANDEDNESS : | double |
| TOPOLOGY : | linear |
| MOLECULAR TYPE : | synthetic DNA |
| ORIGINAL SOURCE : | |
| ORGANISM : | human |
| IMMEDIATE EXPERIMENTAL SOURCE : | |
| NAME OF CELL-LINE : | |
| FEATURES : | from 1 to 1359 |
| PROPERTIES : | synthesised fragment C DNA increased (G+C)-content |

```
ATG AAA AAC CTT GAT TGT TGG GTC GAC AAC GAA GAA GAC ATC GAT GTT    48
Met Lys Asn Leu Asp Cys Trp Val Asp Asn Glu Glu Asp Ile Asp Val
 1               5                  10                  15

ATC CTG AAA AAG TCT ACC ATT CTG AAC TTG GAC ATC AAC AAC GAT ATT    96
Ile Leu Lys Lys Ser Thr Ile Leu Asn Leu Asp Ile Asn Asn Asp Ile
            20                  25                  30

ATC TCC GAC ATC TCT GGT TTC AAC TCC TCT GTT ATC ACA TAT CCA GAT   144
Ile Ser Asp Ile Ser Gly Phe Asn Ser Ser Val Ile Thr Tyr Pro Asp
        35                  40                  45

GCT CAA TTG GTG CCG GGC ATC AAC GGC AAA GCT ATC CAC CTG GTT AAC   192
Ala Gln Leu Val Pro Gly Ile Asn Gly Lys Ala Ile His Leu Val Asn
    50                  55                  60

AAC GAA TCT TCT GAA GTT ATC GTG CAC AAG GCC ATG GAC ATC GAA TAC   240
Asn Glu Ser Ser Glu Val Ile Val His Lys Ala Met Asp Ile Glu Tyr
65                  70                  75                      80

AAC GAC ATG TTC AAC AAC TTC ACC GTT AGC TTC TGG CTG CGC GTT CCG   288
Asn Asp Met Phe Asn Asn Phe Thr Val Ser Phe Trp Leu Arg Val Pro
                85                  90                  95

AAA GTT TCT GCT TCC CAC CTG GAA CAG TAC GGC ACT AAC GAG TAC TCC   336
Lys Val Ser Ala Ser His Leu Glu Gln Tyr Gly Thr Asn Glu Tyr Ser
            100                 105                 110

ATC ATC AGC TCT ATG AAG AAA CAC TCC CTG TCC ATC GGC TCT GGT TGG   384
Ile Ile Ser Ser Met Lys Lys His Ser Leu Ser Ile Gly Ser Gly Trp
        115                 120                 125

TCT GTT TCC CTG AAG GGT AAC AAC CTG ATC TGG ACT CTG AAA GAC TCC   432
Ser Val Ser Leu Lys Gly Asn Asn Leu Ile Trp Thr Leu Lys Asp Ser
    130                 135                 140

GCG GGC GAA GTT CGT CAG ATC ACT TTC CGC GAC CTG CCG GAC AAG TTC   480
Ala Gly Glu Val Arg Gln Ile Thr Phe Arg Asp Leu Pro Asp Lys Phe
145                 150                 155                     160

AAC GCG TAC CTG GCT AAC AAA TGG GTT TTC ATC ACT ATC ACT AAC GAT   528
Asn Ala Tyr Leu Ala Asn Lys Trp Val Phe Ile Thr Ile Thr Asn Asp
                165                 170                 175

CGT CTG TCT TCT GCT AAC CTG TAC ATC AAC GGC GTT CTG ATG GGC TCC   576
Arg Leu Ser Ser Ala Asn Leu Tyr Ile Asn Gly Val Leu Met Gly Ser
            180                 185                 190

GCT GAA ATC ACT GGT CTG GGC GCT ATC CGT GAG GAC AAC AAC ATC ACT   624
Ala Glu Ile Thr Gly Leu Gly Ala Ile Arg Glu Asp Asn Asn Ile Thr
        195                 200                 205

CTT AAG CTG GAC CGT TGC AAC AAC AAC AAC CAG TAC GTA TCC ATC GAC   672
Leu Lys Leu Asp Arg Cys Asn Asn Asn Asn Gln Tyr Val Ser Ile Asp
    210                 215                 220

AAG TTC CGT ATC TTC TGC AAA GCA CTG AAC CCG AAA GAG ATC GAA AAA   720
Lys Phe Arg Ile Phe Cys Lys Ala Leu Asn Pro Lys Glu Ile Glu Lys
225                 230                 235                     240
```

```
CTG TAT ACC AGC TAC CTG TCT ATC ACC TTC CTG CGT GAC TTC TGG GGT    768
Leu Tyr Thr Ser Tyr Leu Ser Ile Thr Phe Leu Arg Asp Phe Trp Gly
                245                 250                 255

AAC CCG CTG CGT TAC GAC ACC GAA TAT TAC CTG ATC CCG GTA GCT TCT    816
Asn Pro Leu Arg Tyr Asp Thr Glu Tyr Tyr Leu Ile Pro Val Ala Ser
            260                 265                 270

AGC TCT AAA GAC GTT CAG CTG AAA AAC ATC ACT GAC TAC ATG TAC CTG    864
Ser Ser Lys Asp Val Gln Leu Lys Asn Ile Thr Asp Tyr Met Tyr Leu
        275                 280                 285

ACC AAC GCG CCG TCC TAC ACT AAC GGT AAA CTG AAC ATC TAC TAC CGA    912
Thr Asn Ala Pro Ser Tyr Thr Asn Gly Lys Leu Asn Ile Tyr Tyr Arg
    290                 295                 300

CGT CTG TAC AAC GGC CTG AAA TTC ATC ATC AAA CGC TAC ACT CCG AAC    960
Arg Leu Tyr Asn Gly Leu Lys Phe Ile Ile Lys Arg Tyr Thr Pro Asn
305                 310                 315                 320

AAC GAA ATC GAT TCT TTC GTT AAA TCT GGT GAC TTC ATC AAA CTG TAC   1008
Asn Glu Ile Asp Ser Phe Val Lys Ser Gly Asp Phe Ile Lys Leu Tyr
                325                 330                 335

GTT TCT TAC AAC AAC AAC GAA CAC ATC GTT GGT TAC CCG AAA GAC GGT   1056
Val Ser Tyr Asn Asn Asn Glu His Ile Val Gly Tyr Pro Lys Asp Gly
            340                 345                 350

AAC GCT TTC AAC AAC CTG GAC AGA ATT CTG CGT GTT GGT TAC AAC GCT   1104
Asn Ala Phe Asn Asn Leu Asp Arg Ile Leu Arg Val Gly Tyr Asn Ala
        355                 360                 365

CCG GGT ATC CCG CTG TAC AAA AAA ATG GAA GCT GTT AAA CTG CGT GAC   1152
Pro Gly Ile Pro Leu Tyr Lys Lys Met Glu Ala Val Lys Leu Arg Asp
    370                 375                 380

CTG AAA ACC TAC TCT GTT CAG CTG AAA CTG TAC GAC GAC AAA AAC GCT   1200
Leu Lys Thr Tyr Ser Val Gln Leu Lys Leu Tyr Asp Asp Lys Asn Ala
385                 390                 395                 400

TCT CTG GGT CTG GTT GGT ACC CAC AAC GGT CAG ATC GGT AAC GAC CCG   1248
Ser Leu Gly Leu Val Gly Thr His Asn Gly Gln Ile Gly Asn Asp Pro
                405                 410                 415

AAC CGT GAC ATC CTG ATC GCT TCT AAC TGG TAC TTC AAC CAC CTG AAA   1296
Asn Arg Asp Ile Leu Ile Ala Ser Asn Trp Tyr Phe Asn His Leu Lys
            420                 425                 430

GAC AAA ATC CTG GGT TGC GAC TGG TAC TTC GTT CCG ACC GAT GAA GGT   1344
Asp Lys Ile Leu Gly Cys Asp Trp Tyr Phe Val Pro Thr Asp Glu Gly
        435                 400                 445

TGG ACC AAC GAC TAA                                               1359
Trp Thr Asn Asp ***
    450
```

## SEQUENCE LISTING

| | |
|---|---|
| SEQ ID NO : | 3 |
| SEQUENCE TYPE : | nucleotide |
| SEQUENCE LENGTH : | 310 bases |
| STRANDEDNESS : | double |
| TOPOLOGY : | linear |
| MOLECULAR TYPE : | synthetic |
| ORIGINAL SOURCE : | genomic DNA |
| ORGANISM : | - |
| IMMEDIATE EXPERIMENTAL SOURCE : | - |
| NAME OF CELL-LINE : | - |
| FEATURES : | from 1 to 310 bp |
| PROPERTIES : | promoter region of GAL 7 |

60

CTCGAGACGT CTATACTTCG GAGCACTGTT GAGCGAAGGC TCATTAGATA TATTTTCTGT
GAGCTCTGCA GATATGAAGC CTCGTGACAA CTCGGTTCCG AGTAATCTAT ATAAAAGACA

120

CATTTTCCTT AACCCAAAAA TAAGGGAGAG GGTCCAAAAA GCGCTCGGAC AACTGTTGAC
GTAAAAGGAA TTGGGTTTTT ATTCCCTCTC CCAGGTTTTT CGCGAGCCTG TTGACAACTG

180

CGTGATCCGA AGGACTGGCT ATACAGTGTT CACAAAATAG CCAAGCTGAA AATAATGTGT
GCACTAGGCT TCCTGACCGA TATGTCACAA GTGTTTTATC GGTTCGACTT TTATTACACA

240

AGCCTTTAGC TATGTTCAGT TAGTTTGGCT AGCAAAGATA TAAAAGCAGG TCGGAAATAT
TCGGAAATCG ATACAAGTCA ATCAAACCGA TCGTTTCTAT ATTTTCGTCC AGCCTTTATA

300

TTATGGGCAT TATTATGCAG AGGATCCACA TGATAAAAAA AACAGTTGAA TATTCCCTCA
AATACCCGTA ATAATACGTC TCCTAGGTGT ACTATTTTTT TTGTCAACTT ATAAGGGAGT

310

AAAATGACTG
TTTTACTGAC

SEQUENCE LISTING

| | |
|---|---|
| SEQ ID NO : | 4 |
| SEQUENCE TYPE : | nucleotide |
| SEQUENCE LENGTH : | 32 bases |
| STRANDEDNESS : | double (sticky ends) |
| TOPOLOGY : | linear |
| MOLECULAR TYPE : | synthetic |
| ORIGINAL SOURCE : | |
| ORGANISM : | |
| IMMEDIATE EXPERIMENTAL SOURCE : | |
| NAME OF CELL-LINE : | |
| FEATURES : | from 1 to 32 |
| PROPERTIES : | acts as a junction between yeast promoter and inserted gene |

GATCTAAACG ATGAAAAACC TTGATTGTTG GG 32

SEQUENCE LISTING

| | |
|---|---|
| SEQ ID NO : | 5 |
| SEQUENCE TYPE : | nucleotide |
| SEQUENCE LENGTH : | 32 bases |
| STRANDEDNESS : | double (sticky ends) |
| TOPOLOGY : | linear |
| MOLECULAR TYPE : | synthetic |
| ORIGINAL SOURCE : | |
| ORGANISM : | |
| IMMEDIATE EXPERIMENTAL SOURCE : | |
| NAME OF CELL-LINE : | |
| FEATURES : | from 1 to 32 |
| PROPERTIES : | acts as a junction between yeast promoter and inserted gene |

TCGACCCAAC AATCAAGGTT TTTCATCGTT TA 32

SEQUENCE LISTING

SEQ ID NO : 6

SEQUENCE TYPE : nucleotide

SEQUENCE LENGTH : 63 bases

STRANDEDNESS : double (sticky ends)

TOPOLOGY : linear

MOLECULAR TYPE : synthetic

ORIGINAL SOURCE :

ORGANISM :

IMMEDIATE EXPERIMENTAL SOURCE :

NAME OF CELL-LINE :

FEATURES : from 1 to 63

PROPERTIES : acts as a junction between yeast promoter and inserted gene

GATCTACATG ATAAAAAAAA GAGTTGAATA TTCCCTCAAC CATGGTTAAC TTGGACTGTT 60
GGG 63

SEQUENCE LISTING

| | |
|---|---|
| SEQ ID NO : | 7 |
| SEQUENCE TYPE : | nucleotide |
| SEQUENCE LENGTH : | 63 bases |
| STRANDEDNESS : | double (sticky ends) |
| TOPOLOGY : | linear |
| MOLECULAR TYPE : | synthetic |
| ORIGINAL SOURCE : | |
| ORGANISM : | |
| IMMEDIATE EXPERIMENTAL SOURCE : | |
| NAME OF CELL-LINE : | |
| FEATURES : | from 1 to 63 |
| PROPERTIES : | acts as a junction between yeast promoter and inserted gene |

```
TCGACCCAAC AGTCCAAGTT AACCATGGTT GAGGGAATAT TCAACTGTTT TTTTTATCAT   60
GTA                                                                 63
```

SEQUENCE LISTING

SEQ ID NO : 8

SEQUENCE TYPE : Nucleotide with corresponding protein

SEQUENCE LENGTH : 312 bases

STRANDEDNESS : Single

TOPOLOGY : Linear

MOLECULAR TYPE : Synthetic

ORIGINAL SOURCE :

ORGANISM :

IMMEDIATE EXPERIMENTAL SOURCE :

NAME OF CELL-LINE :

FEATURES : from 1 to 312

PROPERTIES :

```
GATCTACATG  ATAAAAAAAA  CAGTTGAATA  TTCCCTCAAA  A  ATG AGA TTT CCT TCA ATT      59
                                                   Met Arg Phe Pro Ser Ile
                                                    1               5

TTT ACT GCA GTT TTA TTC GCA GCA TCC TCC GCA TTA GCT GCT CCA GTC               107
Phe Thr Ala Val Leu Phe Ala Ala Ser Ser Ala Leu Ala Ala Pro Val
            10                  15                  20
```

```
AAC ACT ACA ACA GAA GAT GAA ACG GCA CAA ATT CCG GCT GAA GCT GTC      155
Asn Thr Thr Thr Glu Asp Glu Thr Ala Gln Ile Pro Ala Glu Ala Val
         25                  30                  35

ATC GGT TAC TCA GAT TTA GAA GGG GAT TTC GAT GTT GCT GTT TTG CCA      203
Ile Gly Tyr Ser Asp Leu Glu Gly Asp Phe Asp Val Ala Val Leu Pro
     40                  45                  50

TTT TCC AAC AGC ACA AAT AAC GGG TTA TTG TTT ATA AAT ACT ACT ATT      251
Phe Ser Asn Ser Thr Asn Asn Gly Leu Leu Phe Ile Asn Thr Thr Ile
 55                  60                  65                  70

GCC AGC ATT GCT GCT AAA GAA GAA GGG GTA TCT CTC GAG AAA AGA GAG      299
Ala Ser Ile Ala Ala Lys Glu Glu Gly Val Ser Leu Glu Lys Arg Glu
                 75                  80                  85

GCT GAA GCC ATG G                                                    312
Ala Glu Ala Met
            90
```

## SEQUENCE LISTING

SEQ ID NO : 9

SEQUENCE TYPE : nucleotide

SEQUENCE LENGTH : 75 bases

STRANDEDNESS : double (sticky ends)

TOPOLOGY : linear

MOLECULAR TYPE : synthetic

ORIGINAL SOURCE :

ORGANISM :

IMMEDIATE EXPERIMENTAL SOURCE :

NAME OF CELL-LINE :

FEATURES : from 1 to 75 bp

PROPERTIES :

```
CGAAGGATCC AAACGATGAG ATTTCCTTCA ATTTTTACTG CAGACTAGTC CCGGGTAAGT 60
AAGTAAGCGG CCGCG                                                  75
```

## SEQUENCE LISTING

| | |
|---|---|
| SEQ ID NO : | 10 |
| SEQUENCE TYPE : | nucleotide |
| SEQUENCE LENGTH : | 77 bases |
| STRANDEDNESS : | double (sticky ends) |
| TOPOLOGY : | linear |
| MOLECULAR TYPE : | synthetic |
| ORIGINAL SOURCE : | |
| ORGANISM : | |
| IMMEDIATE EXPERIMENTAL SOURCE : | |
| NAME OF CELL-LINE : | |
| FEATURES : | from 1 to 77 bp |
| PROPERTIES : | |

```
AATTCGCGGC CGCTTACTTA CTTACCCGGG ACTAGTCTGC AGTAAAAATT GAAGGAAATC 60
TCATCGTTTG GATCCTT                                                77
```

## Claims

### Claims for the following Contracting States : AT, BE, CH, DE, DK, FR, GB, IT, LI, LU, NL, SE

1. A DNA sequence encoding tetanus toxin fragment C and having a (G+C)-content that has been increased in the region from nucleotide 410 to the 3' end of the coding sequence relative to the wild-type DNA sequence so as to allow the production of complete mRNA transcripts in yeast, the nucleotide numbering corresponding to that set forth in SEQ ID NOS: 1 and 2.

2. A DNA sequence according to claim 1, wherein the said (G+C)-content has been increased in each of the following regions:
   (i) from nucleotide 510 to nucleotide 710,
   (ii) from nucleotide 650 to nucleotide 850,
   (iii) from nucleotide 800 to nucleotide 1100,
   (iv) from nucleotide 900 to nucleotide 1200 and
   (v) from nucleotide 1100 to the 3' end of the coding sequence.

3. A DNA sequence according to claim 2, wherein the said (G+C)-content has also been increased in the region:
   (vi) from nucleotide 410 to nucleotide 610.

4. A DNA sequence according to any one of claims 1 to 3, wherein the said (G+C)-content is from 40 to 60 %.

5. A DNA sequence according to any one of claims 1 to 4, substantially as shown in SEQ ID NO: 2.

6. An expression vector which incorporates a DNA sequence according to any one of claims 1 to 5 and which is capable of expressing fragment C in yeast.

7. A vector according to claim 6, which is an autonomously replicating plasmid.

8. A yeast organism transformed with a vector according to claim 6 or 7.

9. A transformed yeast organism according to claim 8, wherein the yeast is Saccharomyces cerevisiae.

10. A transformed yeast organism according to claim 8, wherein the yeast is Pichia pastoris.

11. A process for the preparation of fragment C of tetanus toxin, which comprises culturing a transformed yeast organism according to any one of claims 8 to 10 and recovering the product fragment C thus expressed.

12. A process according to claim 11, which further comprises formulating the fragment C thus recovered with a pharmaceutically acceptable carrier or diluent to form thereby a vaccine.

### Claims for the following Contracting State : ES

1. A process for the preparation of fragment C of tetanus toxin, which process comprises culturing a yeast organism transformed with an expression vector which incorporates a DNA sequence encoding tetanus toxin fragment C and having a (G+C)-content that has been increased in the region from nucleotide 410 to the 3' end of the coding sequence relative to the wild-type DNA sequence so as to allow the production of complete mRNA transcripts in yeast, the nucleotide numbering corresponding to that set forth in SEQ ID NOS: 1 and 2; and recovering the product fragment C thus expressed.

2. A process according to claim 1, wherein the said (G+C)-content has been increased in each of the following regions:
   (i) from nucleotide 510 to nucleotide 710,
   (ii) from nucleotide 650 to nucleotide 850,
   (iii) from nucleotide 800 to nucleotide 1100,
   (iv) from nucleotide 900 to nucleotide 1200 and
   (v) from nucleotide 1100 to the 3' end of the coding sequence.

3. A process according to claim 2, wherein the said (G+C)-content has also been increased in the region:
   (vi) from nucleotide 410 to nucleotide 610.

4. A process according to any one of claims 1 to 3, wherein the said (G+C)-content is from 40 to 60 %.

5. A process according to any one of claims 1 to 4, wherein the said DNA is substantially as shown in SEQ ID NO: 2.

6. A process according to any one of claims 1 to 5, wherein the expression vector is an autonomously replicating plasmid.

7. A process according to any one of claims 1 to 6, wherein the yeast is Saccharomyces cerevisiae.

8. A process according to any one of claims 1 to 6, wherein the yeast is Pichia pastoris.

9. A process according to any one of claims 1 to 8, which further comprises formulating the fragment C thus recovered with a pharmaceutically acceptable carrier or diluent to form thereby a vaccine.

**Patentansprüche**

**Patentansprüche für folgende Vertragsstaaten : AT, BE, CH, DE, DK, FR, GB, IT, LI, LU, NL, SE**

1. DNA-Sequenz, die für Fragment C des Tetanus-Toxins codiert und einen (G+C)-Gehalt aufweist, der in der Region von Nucleotid 410 bis zum 3'-Ende der codierenden Sequenz relativ zur Wildtyp-DNA-Sequenz erhöht worden ist, so daß die Bildung vollständiger mRNA-Transkripte in Hefe ermöglicht wird, wobei die Numerierung der Nucleotide der bei den Sequenzen Nr. 1 und 2 angegebenen Numerierung entspricht.

2. DNA-Sequenz nach Anspruch 1, wobei der (G+C)-Gehalt in der. nachstehenden Regionen erhöht worden ist:
   (i) von Nucleotid 510 bis Nucleotid 710,
   (ii) von Nucleotid 650 bis Nucleotid 850,
   (iii) von Nucleotid 800 bis Nucleotid 1100,
   (iv) von Nucleotid 900 bis Nucleotid 1200 und
   (v) von Nucleotid 1100 bis zum 3'-Ende der codierenden Sequenz.

3. DNA-Sequenz nach Anspruch 2, wobei der (G+C)-Gehalt auch in der folgenden Region erhöht worden ist:
   (vi) von Nucleotid 410 bis Nucleotid 610.

4. DNA-Sequenz nach einem der Ansprüche 1 bis 3, wobei der (G+C)-Gehalt von 40 bis 60 % beträgt.

5. DNA-Sequenz nach einem der Ansprüche 1 bis 4, im wesentlichen wie bei Sequenz Nr. 2 gezeigt.

6. Expressionsvektor, der eine DNA-Sequenz gemäß einem der Ansprüche 1 bis 5 enthält und zur Expression von Fragment C in Hefe fähig ist.

7. Expressionsvektor nach Anspruch 6, wobei es sich um ein sich autonom replizierendes Plasmid handelt.

8. Hefeorganismus, der mit einem Vektor nach Anspruch 6 oder 7 transformiert ist.

9. Transformierter Hefeorganismus nach Anspruch 8, wobei es sich bei der Hefe um Saccharomyces cerevisiae handelt.

10. Transformierter Hefeorganismus nach Anspruch 8, wobei es sich bei der Hefe um Pichia pastoris handelt.

11. Verfahren zur Herstellung von Fragment C des Tetanus-Toxins, das die Züchtung eines transformierten Hefeorganismus nach einem der Ansprüche 8 bis 10 und die Gewinnung des auf diese Weise exprimierten Fragments C als Produkt umfaßt.

12. Verfahren nach Anspruch 11, das ferner die Formulierung des auf diese Weise gewonnenen Fragments C mit einem pharmazeutisch verträglichen Träger oder Verdünnungsmittel umfaßt, um dadurch ein Vakzin zu bilden.

**Patentansprüche für folgenden Vertragstaat : ES**

1. Verfahren zur Herstellung von Fragment C des Tetanus-Toxins, das die Züchtung eines transformierten Hefeorganismus mit einem Vektor, der eine DNA-Sequenz enthält, die für Fragment C des Tetanus-Toxins codiert und einen (G+C)-Gehalt aufweist, der in der Region von Nucleotid 410 bis zum 3'-Ende der codierenden Sequenz relativ zur Wildtyp-DNA-Sequenz erhöht worden ist, so daß die Bildung vollständiger mRNA-Transkripte in Hefe ermöglicht wird, wobei die Numerierung der Nucleotide der bei den Sequenzen Nr. 1 und 2 angegebenen Numerierung entspricht, und die Gewinnung des auf diese Weise exprimierten Fragments C als Produkt umfaßt.

2. Verfahren nach Anspruch 1, wobei der (G+C)-Gehalt in den nachstehenden Regionen erhöht worden ist:
   (i) von Nucleotid 510 bis Nucleotid 710,
   (ii) von Nucleotid 650 bis Nucleotid 850,
   (iii) von Nucleotid 800 bis Nucleotid 1100,
   (iv) von Nucleotid 900 bis Nucleotid 1200 und
   (v) von Nucleotid 1100 bis zum 3'-Ende der codierenden Sequenz.

3. Verfahren nach Anspruch 2, wobei der (G+C)-Gehalt auch in der folgenden Region erhöht worden ist:
   (vi) von Nucleotid 410 bis Nucleotid 610.

4. Verfahren nach einem der Ansprüche 1 bis 3, wobei der (G+C)-Gehalt von 40 bis 60 % beträgt.

5. Verfahren nach einem der Ansprüche 1 bis 4, wobei die DNA im wesentlichen wie bei Sequenz Nr. 2 gezeigt ist.

6. Verfahren nach einem der Ansprüche 1 bis 5, wobei es sich bei dem Expressionsvektor um ein sich autonom replizierendes Plasmid handelt.

7. Verfahren nach einem der Ansprüche 1 bis 6, wobei es sich bei der Hefe um Saccharomyces cerevisiae handelt.

8. Verfahren nach einem der Ansprüche 1 bis 6, wobei es sich bei der Hefe um Pichia pastoris handelt.

9. Verfahren nach einem der Ansprüche 1 bis 8, das ferner die Formulierung des auf diese Weise gewonnenen Fragments C mit einem pharmazeutisch verträglichen Träger oder Verdünnungsmittel umfaßt, um dabei ein Vakzin zu bilden.

**Revendications**

**Revendications pour les Etats contractants suivants : DE, GB, FR, IT, NL, SE, LI, CH, BE, AT, LU, DK**

1. Séquence d'ADN codant pour le fragment C de la toxine tétanique et ayant une teneur accrue en (G+C) qui a été augmentée dans la région du nucléotide 410 jusqu'à l'extrémité 3' terminale de la région codante par rapport à la séquence d'ADN du type sauvage de façon à permettre la production de transcrits d'ARN complets dans une levure, la numérotation des nucléotides correspondant à celle indiquée dans SEQ ID n° 1 et 2.

2. Séquence d'ADN suivant la revendication 1, dans laquelle la teneur en (G+C) a été augmentée dans chacune des régions suivantes :
   (i) du nucléotide 510 au nucléotide 710,
   (ii) du nucléotide 650 au nucléotide 850,
   (iii) du nucléotide 800 au nucléotide 1100,
   (iv) du nucléotide 900 au nucléotide 1200, et

(v) du nucléotide 1100 à l'extrémité 3' terminale de la séquence codante.

3. Séquence d'ADN suivant la revendication 2, dans laquelle la teneur en (G+C) a aussi été augmentée dans la région :
   (vi) du nucléotide 410 au nucléotide 610.

4. Séquence d'ADN suivant l'une quelconque des revendications 1 à 3, dans laquelle la teneur en (G+C) est de 40 à 60%.

5. Séquence d'ADN suivant l'une quelconque des revendications 1 à 4, en substance comme indiqué dans SEQ ID n° 2.

6. Vecteur d'expression qui comprend une séquence d'ADN suivant l'une quelconque des revendications 1 à 5 et qui est capable d'exprimer le fragment C dans une levure.

7. Vecteur suivant la revendication 6, qui est un plasmide à réplication autonome.

8. Organisme de levure transformé par un vecteur suivant la revendication 6 ou 7.

9. Organisme de levure transformé suivant la revendication 8, dans lequel la levure est Saccharomyces cerevisiae.

10. Organisme de levure transformé suivant la revendication 8, dans lequel la levure est Pichia pastoris.

11. Procédé de préparation du fragment C de la toxine tétanique, qui comprend la mise en culture d'un organisme de levure transformé suivant l'une quelconque des revendications 8 à 10 et l'isolement du fragment C produit ainsi exprimé.

12. Procédé suivant la revendication 11, qui comprend de plus la mise en composition du fragment C ainsi isolé avec un excipient ou diluant pharmaceutiquement acceptable pour former un vaccin.

**Revendication pour l'Etat contractant suivant : ES**

1. Procédé de préparation du fragment C de la toxine tétanique, lequel procédé comprend la mise en culture d'un organisme de levure transformé par un vecteur d'expression qui comprend une séquence d'ADN codant pour le fragment C de la toxine tétanique et ayant une teneur accrue en (G+C) qui a été augmentée dans la région du nucléotide 410 jusqu'à l'extrémité 3' terminale de la séquence codante par rapport à la séquence d'ADN du type sauvage de façon à permettre la production de transcrits d'ARNm complets dans une levure, la numérotation des nucléotides correspondant à celle indiquée dans SEQ ID n° 1 et 2, et l'isolement du fragment C produit ainsi exprimé.

2. Procédé suivant la revendication 1, dans lequel la teneur en (G+C) a été augmentée dans chacune des régions suivantes :
   (i) du nucléotide 510 au nucléotide 710,
   (ii) du nucléotide 650 au nucléotide 850,
   (iii) du nucléotide 800 au nucléotide 1100,
   (iv) du nucléotide 900 au nucléotide 1200, et
   (v) du nucléotide 1100 à l'extrémité 3' terminale de la séquence codante.

3. Procédé suivant la revendication 2, dans lequel la teneur en (G+C) a aussi été augmentée dans la région :
   (vi) du nucléotide 410 au nucléotide 610.

4. Procédé suivant l'une quelconque des revendications 1 à 3, dans lequel la teneur en (G+C) est de 40 à 60%.

5. Procédé suivant l'une quelconque des revendications 1 à 4, dans lequel l'ADN est en substance tel qu'indiqué dans SEQ ID n° 2.

6. Procédé suivant l'une quelconque des revendications 1 à 5, dans lequel le vecteur d'expression est un plasmide à réplication autonome.

7. Procédé suivant l'une quelconque des revendications 1 à 6, dans lequel la levure est Saccharomyces cerevisiae.

8. Procédé suivant l'une quelconque des revendications 1 à 6, dans lequel la levure est Pichia pastoris.

9. Procédé suivant l'une quelconque des revendications 1 à 8, qui comprend de plus la mise en composition du fragment C ainsi isolé avec un excipient ou diluant pharmaceutiquement acceptable pour former un vaccin.

FIG.1

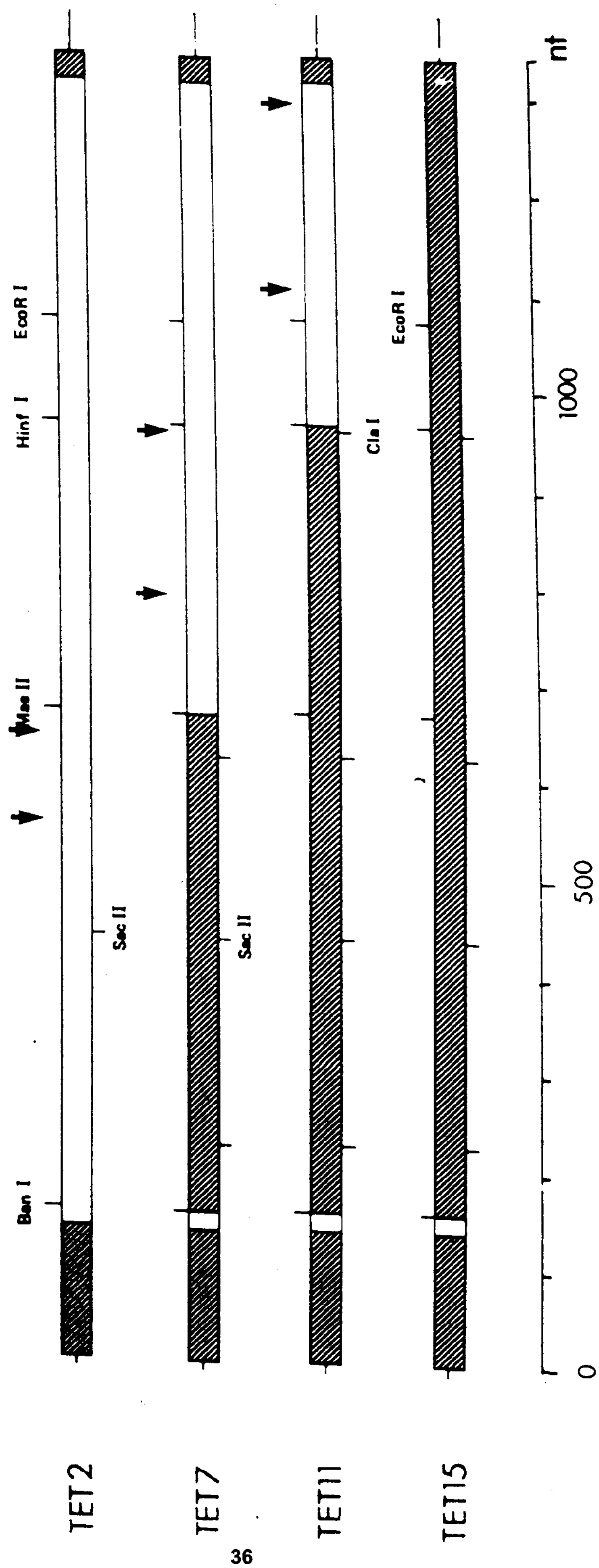
TET2
TET7
TET11
TET15
EcoR I
Hinf I
Mae II
Sac II
Ban I
Cla I
EcoR I
Sac II
0
500
1000
nt

```
                                    30                                      60                                       90
ATG AAA AAT CTG GAT TGT TGG GTT GAT AAT GAA GAA GAT ATA GAT GTT ATA TTA AAA AAG AGT ACA ATT TTA AAT TTA GAT ATT AAT AAT
          C   T               C   C   C           C   C           C C G         TC    C     C G   C   G   C   C   C   C
Met Lys Asn Leu Asp Cys Trp Val Asp Asn Glu Glu Asp Ile Asp Val Ile Leu Lys Lys Ser Thr Ile Leu Asn Leu Asp Ile Asn Asn

                                   120                                     150                                      180
GAT ATT ATA TCA GAT ATA TCT GGG TTT AAT TCA TCT GTA ATA ACA TAT CCA GAT GCT CAA TTG GTG CCC GGA ATA AAT GGC AAA GCA ATA
          C   C   C   C       T   C   C   C       T   C                                   G   C   C   C           T   C
Asp Ile Ile Ser Asp Ile Ser Gly Phe Asn Ser Ser Val Ile Thr Tyr Pro Asp Ala Gln Leu Val Pro Gly Ile Asn Gly Lys Ala Ile

                                   210                                     240                                      270
CAT TTA GTA AAC AAT GAA TCT TCT GAA GTT ATA GTG CAT AAA GCT ATG GAT ATT GAA TAT AAT GAT ATG TTT AAT AAT TTT ACC GTT AGC
  C C G   T       C                       C       C   G   C       C   C       C   C   C       C   C   C   C
His Leu Val Asn Asn Glu Ser Ser Glu Val Ile Val His Lys Ala Met Asp Ile Glu Tyr Asn Asp Met Phe Asn Asn Phe Thr Val Ser

                                   300                                     330                                      360
TTT TGG TTG AGG GTT CCT AAA GTA TCT GCT AGT CAT TTA GAA CAA TAT GGC ACA AAT GAG TAT TCA ATA ATT AGC TCT ATG AAA AAA CAT
  C     C   C C       G       T         TCC   C C G       G   C       T   C       C   C   C   C               G       C
Phe Trp Leu Arg Val Pro Lys Val Ser Ala Ser His Leu Glu Gln Tyr Gly Thr Asn Glu Tyr Ser Ile Ile Ser Ser Met Lys Lys His

                                   390                                     420                                      450
AGT CTA TCA ATA GGA TCT GGT TGG AGT GTA TCA CTT AAA GGT AAT AAC TTA ATA TGG ACT TTA AAA GAT TCC GCG GGA GAA GTT AGA CAA
TCC   G   C   C   C             TC    T   C   G   G       C     C G   C         C G       C           C         C T   G
Ser Leu Ser Ile Gly Ser Gly Trp Ser Val Ser Leu Lys Gly Asn Asn Leu Ile Trp Thr Leu Lys Asp Ser Ala Gly Glu Val Arg Gln

                                   480                                     510                                      540
ATA ACT TTT AGG GAT TTA CCT GAT AAA TTT AAT GCT TAT TTA GCA AAT AAA TGG GTT TTT ATA ACT ATT ACT AAT GAT AGA TTA TCT TCT
  C       C C C   C C G   G   C   G   C   C   G   C C G   T   C               C   C       C       C     C T C G
Ile Thr Phe Arg Asp Leu Pro Asp Lys Phe Asn Ala Tyr Leu Ala Asn Lys Trp Val Phe Ile Thr Ile Thr Asn Asp Arg Leu Ser Ser

                                   570                                     600                                      630
GCT AAT TTG TAT ATA AAT GGA GTA CTT ATG GGA AGT GCA GAA ATT ACT GGT TTA GGA GCT ATT AGA GAG GAT AAT AAT ATA ACA TTA AAA
      C C G   C   C   C   C   T   G       C TCC   T       C         C G   C       C C T       C   C   C   C   T C T   G
Ala Asn Leu Tyr Ile Asn Gly Val Leu Met Gly Ser Ala Glu Ile Thr Gly Leu Gly Ala Ile Arg Glu Asp Asn Asn Ile Thr Leu Lys

                                   660                                     690                                      720
CTA GAT AGA TGT AAT AAT AAT AAT CAA TAC GTT TCT ATT GAT AAA TTT AGG ATA TTT TGC AAA GCA TTA AAT CCA AAA GAG ATT GAA AAA
  G   C C T   C   C   C   C   C   G       A   C   C   C   G   C C T   C   C             C G   C   G           C
Leu Asp Arg Cys Asn Asn Asn Asn Gln Tyr Val Ser Ile Asp Lys Phe Arg Ile Phe Cys Lys Ala Leu Asn Pro Lys Glu Ile Glu Lys

                                   750                                     780                                      810
TTA TAC ACA AGT TAT TTA TCT ATA ACC TTT TTA AGA GAC TTC TGG GGA AAC CCT TTA CGA TAT GAT ACA GAA TAT TAT TTA ATA CCA GTA
C G   T   C   C   C C G       C       C C G C T               T       G C G   T   C   C   C           C C G   C   G
Leu Tyr Thr Ser Tyr Leu Ser Ile Thr Phe Leu Arg Asp Phe Trp Gly Asn Pro Leu Arg Tyr Asp Thr Glu Tyr Tyr Leu Ile Pro Val

                                   840                                     870                                      900
GCT TCT AGT TCT AAA GAT GTT CAA TTG AAA AAT ATA ACA GAT TAT ATG TAT TTG ACA AAT GCG CCA TCG TAT ACT AAC GGA AAA TTG AAT
          C           C       G C         C   C   T   C           C C     C   C       G   C   C           T     C     C
Ala Ser Ser Ser Lys Asp Val Gln Leu Lys Asn Ile Thr Asp Tyr Met Tyr Leu Thr Asn Ala Pro Ser Tyr Thr Asn Gly Lys Leu Asn

                                   930                                     960                                      990
ATA TAT TAT AGA AGG TTA TAT AAT GGA CTA AAA TTT ATT ATA AAA AGA TAT ACA CCT AAT AAT GAA ATA GAT TCT TTT GTT AAA TCA GGT
  C   C   C C   C T C G   C   C       G       C   C   C     C C   C   T   G   C   C       C           C           T
Ile Tyr Tyr Arg Arg Leu Tyr Asn Gly Leu Lys Phe Ile Ile Lys Arg Tyr Thr Pro Asn Asn Glu Ile Asp Ser Phe Val Lys Ser Gly

                                   1020                                    1050                                    1080
GAT TTT ATT AAA TTA TAT GTA TCA TAT AAC AAT AAT GAG CAC ATT GTA GGT TAT CCG AAA GAT GGA AAT GCC TTT AAT AAT CTT GAT AGA
  C   C   C     C G   C   T   T   C       C   C   A       C   T       C           C   T   C   T   C   C   C   G   C
Asp Phe Ile Lys Leu Tyr Val Ser Tyr Asn Asn Asn Glu His Ile Val Gly Tyr Pro Lys Asp Gly Asn Ala Phe Asn Asn Leu Asp Arg

                                   1110                                    1140                                    1170
ATT CTA AGA GTA GGT TAT AAT GCC CCA GGT ATC CCT CTT TAT AAA AAA ATG GAA GCA GTA AAA TTG CGT GAT TTA AAA ACC TAT TCT GTA
      G C T   T       C   C   T   G           G   G   C                   T   T     C         C C G           C       T
Ile Leu Arg Val Gly Tyr Asn Ala Pro Gly Ile Pro Leu Tyr Lys Lys Met Glu Ala Val Lys Leu Arg Asp Leu Lys Thr Tyr Ser Val

                                   1200                                    1230                                    1260
CAA CTT AAA TTA TAT GAT GAT AAA AAT GCA TCT TTA GGA CTA GTA GGT ACC CAT AAT GGT CAA ATA GGC AAC GAT CCA AAT AGG GAT ATA
  G   G     C G   C   C   C       C   T     C G   T   G   T           C   C       G   C   T       C   G   C C T   C   C
Gln Leu Lys Leu Tyr Asp Asp Lys Asn Ala Ser Leu Gly Leu Val Gly Thr His Asn Gly Gln Ile Gly Asn Asp Pro Asn Arg Asp Ile

                                   1290                                    1320                                    1350
TTA ATT GCA AGC AAC TGG TAC TTT AAT CAT TTA AAA GAT AAA ATT TTA GGA TGT GAT TGG TAC TTT GTA CCT ACA GAT GAA GGA TGG ACA
G G   C   T TCT               C   C   C C G       C       C C G   T   C   C           C   T   G   C           T       C
Leu Ile Ala Ser Asn Trp Tyr Phe Asn His Leu Lys Asp Lys Ile Leu Gly Cys Asp Trp Tyr Phe Val Pro Thr Asp Glu Gly Trp Thr

AAT GAT TAA
  C   C
Asn Asp ...
```

FIG.2

(ID SEQ NO : 1; ID SEQ NO : 2)

'FIG.3

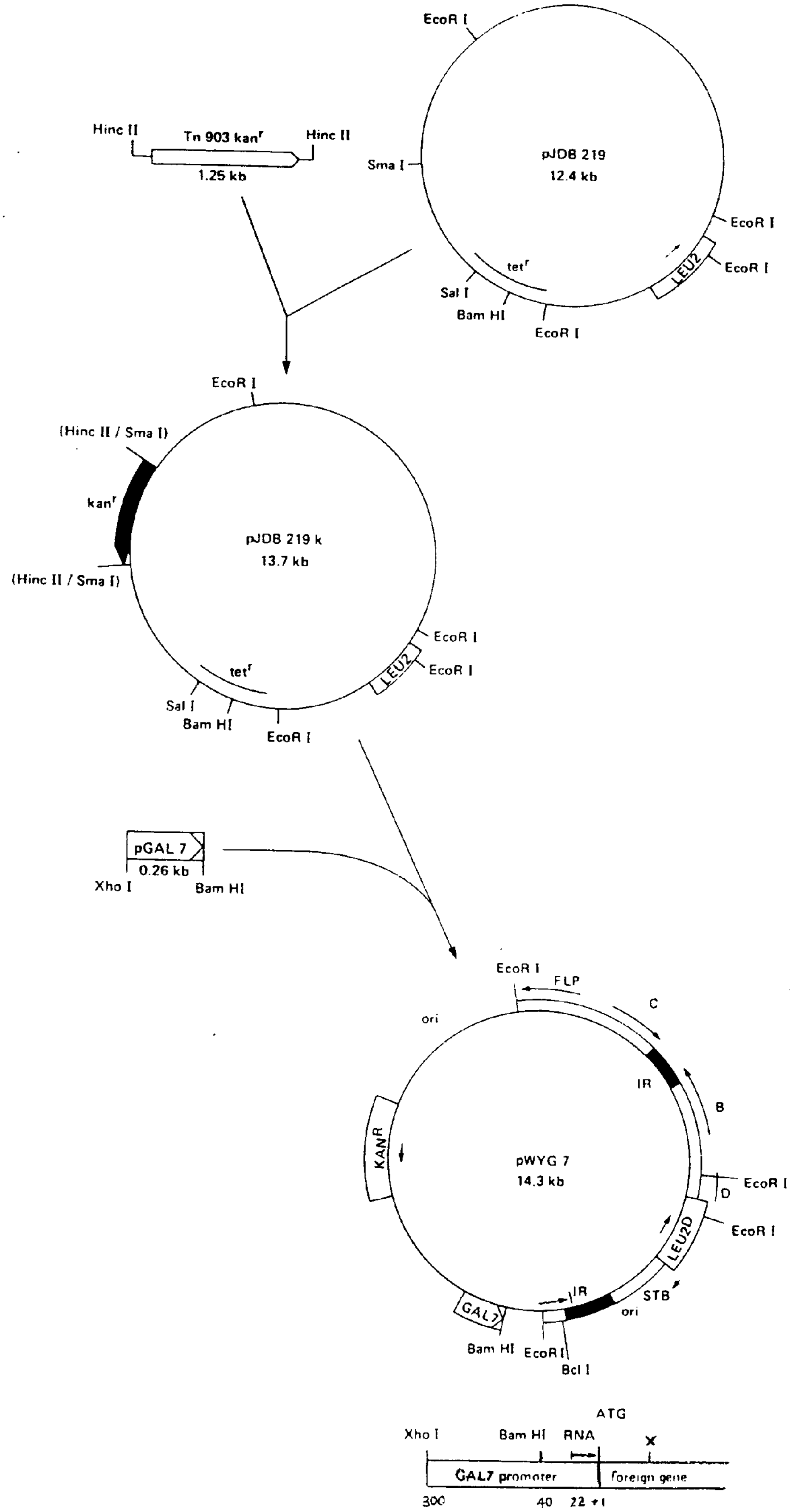
EcoR I
Hinc II
Tn 903 kan$^r$
Hinc II
1.25 kb
Sma I
pJDB 219
12.4 kb
EcoR I
tet$^r$
LEU2
EcoR I
Sal I
Bam HI
EcoR I
EcoR I
(Hinc II / Sma I)
kan$^r$
pJDB 219 k
13.7 kb
(Hinc II / Sma I)
EcoR I
tet$^r$
LEU2
EcoR I
Sal I
Bam HI
EcoR I
pGAL 7
0.26 kb
Xho I
Bam HI
EcoR I
FLP
ori
C
IR
B
KAN$^R$
pWYG 7
14.3 kb
EcoR I
D
EcoR I
LEU2D
IR
STB
ori
GAL7
Bam HI
EcoR I
Bcl I
ATG
Xho I
Bam HI
RNA
X
GAL7 promoter
foreign gene
300
40
22 +1

FIG.4

```
             10         20         30         40         50         60
XhoI
CTCGAGACGT CTATACTTCG GAGCACTGTT GAGCGAAGGC TCATTAGATA TATTTTCTGT
GAGCTCTGCA GATATGAAGC CTCGTGACAA CTCGCTTCCG AGTAATCTAT ATAAAAGACA

             70         80         90        100        110        120
CATTTTCCTT AACCCAAAAA TAAGGGAGAG GGTCCAAAAA GCGCTCGGAC AACTGTTGAC
GTAAAAGGAA TTGGGTTTTT ATTCCCTCTC CCAGGTTTTT CGCGAGCCTG TTGACAACTG

            130        140        150        160        170        180
CGTGATCCGA AGGACTGGCT ATACAGTGTT CACAAAATAG CCAAGCTGAA AATAATGTGT
GCACTAGGCT TCCTGACCGA TATGTCACAA GTGTTTTATC GGTTCGACTT TTATTACACA

            190        200        210        220        230        240
AGCCTTTAGC TATGTTCAGT TAGTTTGGCT AGCAAAGATA TAAAAGCAGG TCGGAAATAT
TCGGAAATCG ATACAAGTCA ATCAAACCGA TCGTTTCTAT ATTTTCGTCC AGCCTTTATA

            250        260        270        280        290        300
                      BamHI                  ↓
TTATGGGCAT TATTATGCAG AGGATCCACA TGATAAAAAA AACAGTTGAA TATTCCCTCA
AATACCCGTA ATAATACGTC TCCTAGGTGT ACTATTTTTT TTGTCAACTT ATAAGGGAGT

            310
AAAATGACTG
TTTTACTGAC
```

(SEQ ID NO : 3)

FIG.5

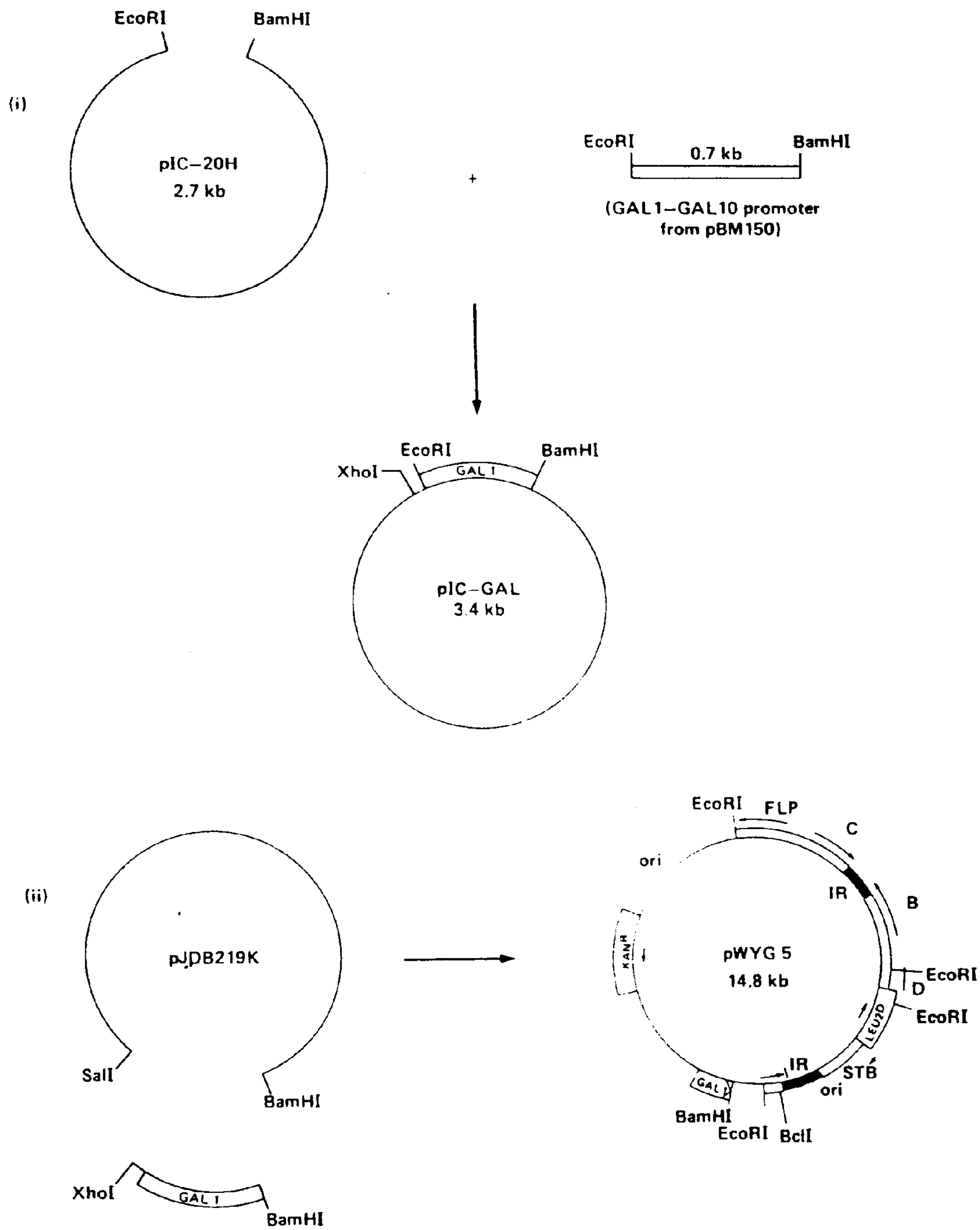
(i)
EcoRI
BamHI
pIC-20H
2.7 kb
+
EcoRI
0.7 kb
BamHI
(GAL1-GAL10 promoter
from pBM150)
XhoI
EcoRI
GAL 1
BamHI
pIC-GAL
3.4 kb
(ii)
pJDB219K
SalI
BamHI
XhoI
GAL 1
BamHI
EcoRI
FLP
C
ori
IR
B
KAN
pWYG 5
14.8 kb
EcoRI
D
EcoRI
LEU2D
IR
STB
ori
GAL 1
BamHI
EcoRI
BclI

FIG.6

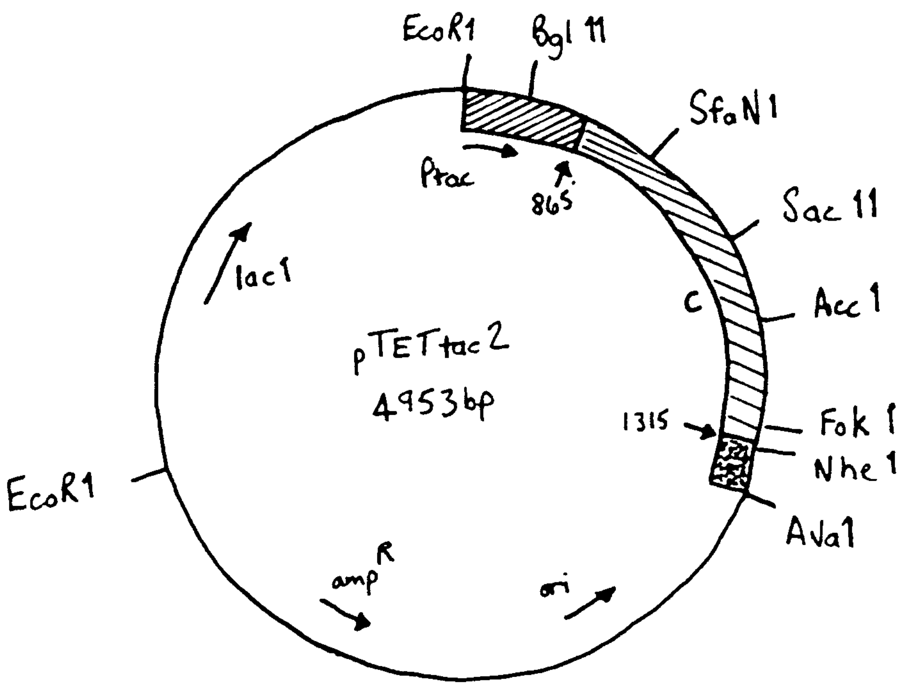
EcoR1
Bgl 11
SfaN1
Sac 11
Acc 1
Fok 1
Nhe 1
Ava1
Ptac
865
C
1315
lac1
pTETtac2
4953bp
EcoR1
amp^R
ori

FIG. 7

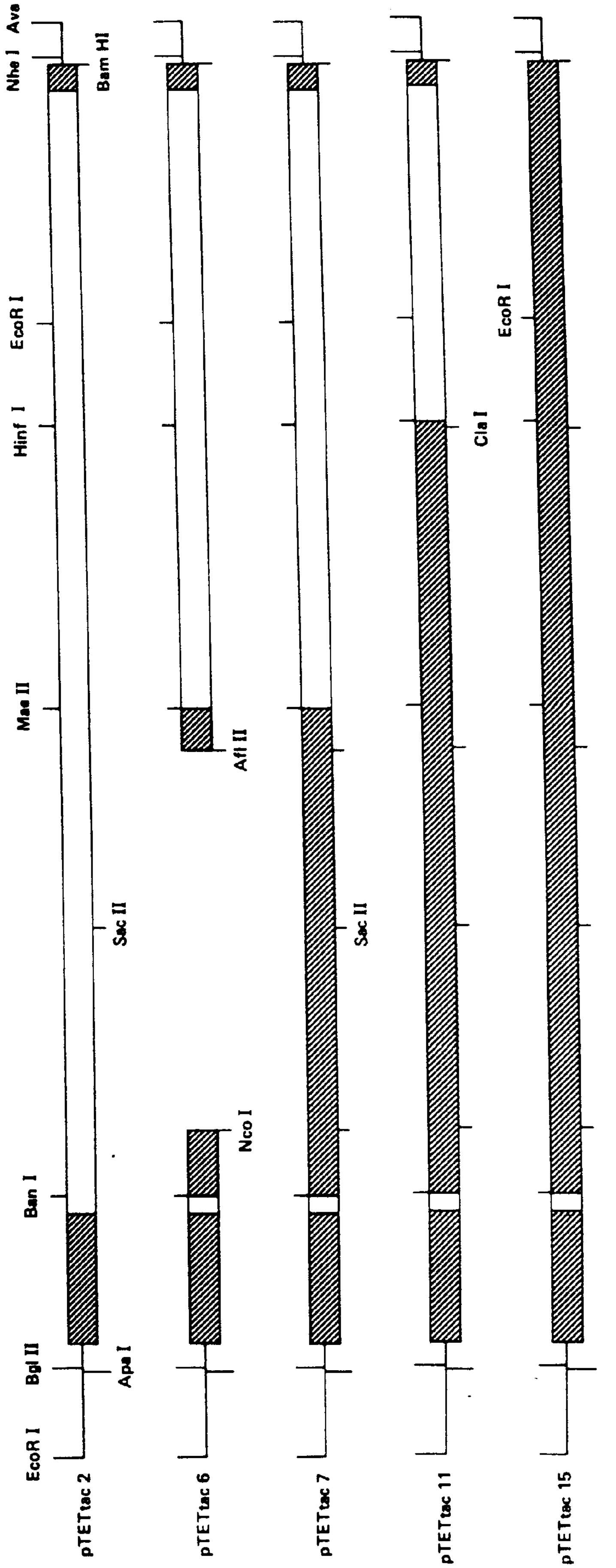
Ava I
Nhe I
Bam HI
EcoR I
Hinf I
Mae II
Sac II
Ban I
Bgl II
Apa I
EcoR I
Afl II
Nco I
Sac II
Cla I
EcoR I
pTETtac 2
pTETtac 6
pTETtac 7
pTETtac 11
pTETtac 15

FIG.8

(i) Construction of pTETtac 14

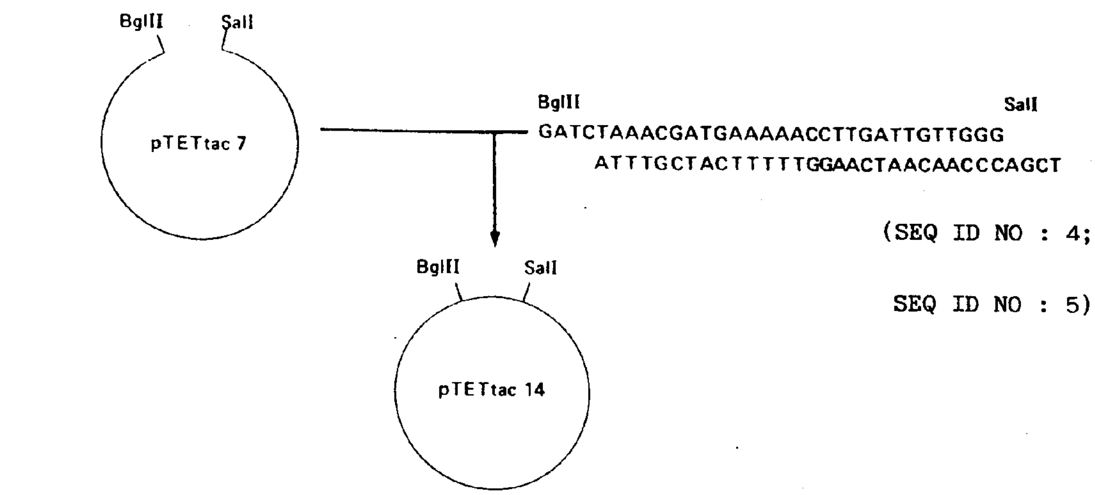


(ii) Construction of pIC–TET

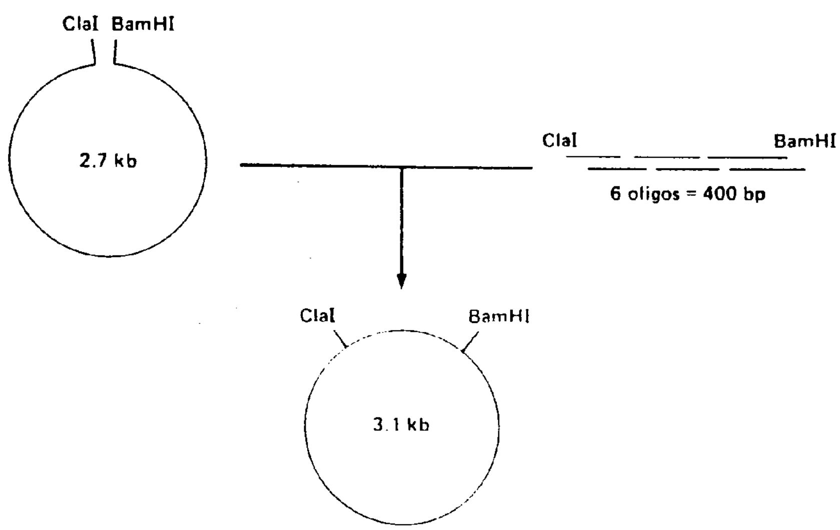


(iii) Construction of pTETtac 16

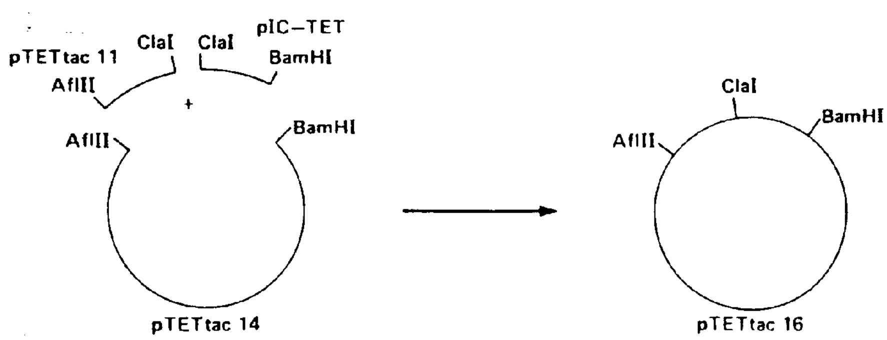

FIG.9

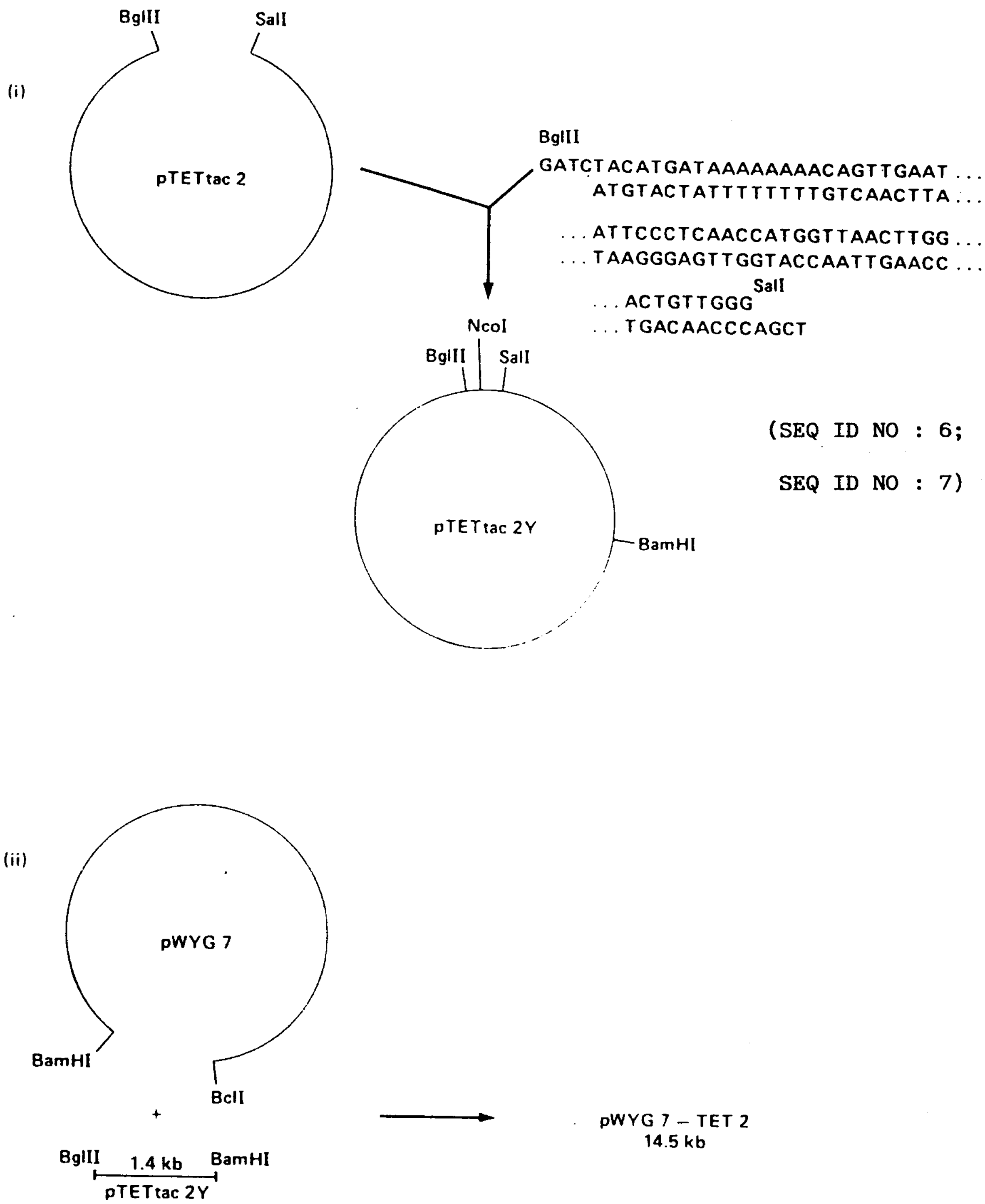
(i)
BglII
SalI
pTETtac 2
BglII
GATCTACATGATAAAAAAAACAGTTGAAT ...
ATGTACTATTTTTTTTGTCAACTTA ...
... ATTCCCTCAACCATGGTTAACTTGG ...
... TAAGGGAGTTGGTACCAATTGAACC ...
SalI
... ACTGTTGGG
... TGACAACCCAGCT
NcoI
BglII
SalI
pTETtac 2Y
BamHI
(SEQ ID NO : 6;
SEQ ID NO : 7)
(ii)
pWYG 7
BamHI
BclI
+
BglII
1.4 kb
BamHI
pTETtac 2Y
pWYG 7 – TET 2
14.5 kb

FIG.10

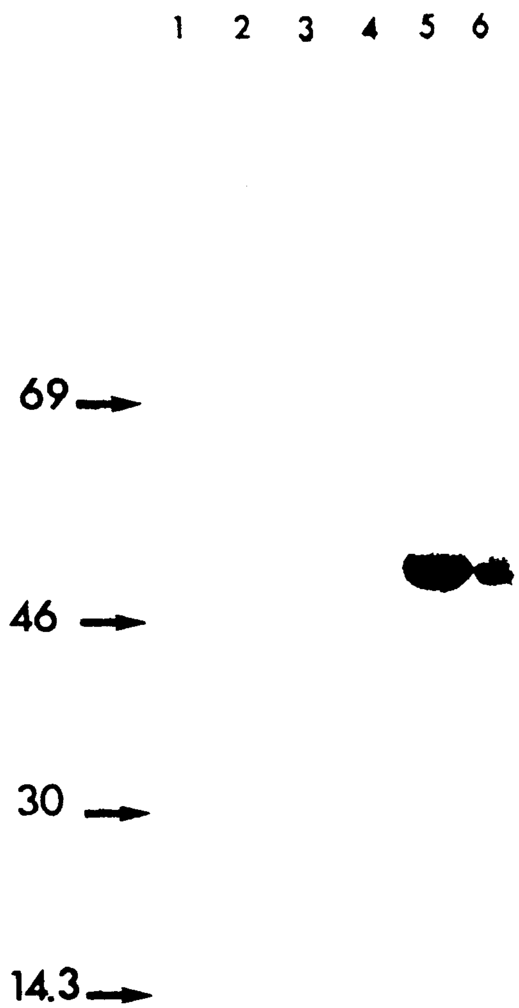

FIG. 11

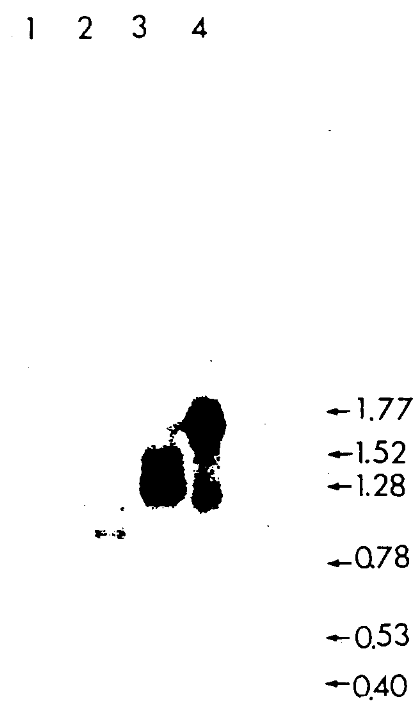

FIG.12

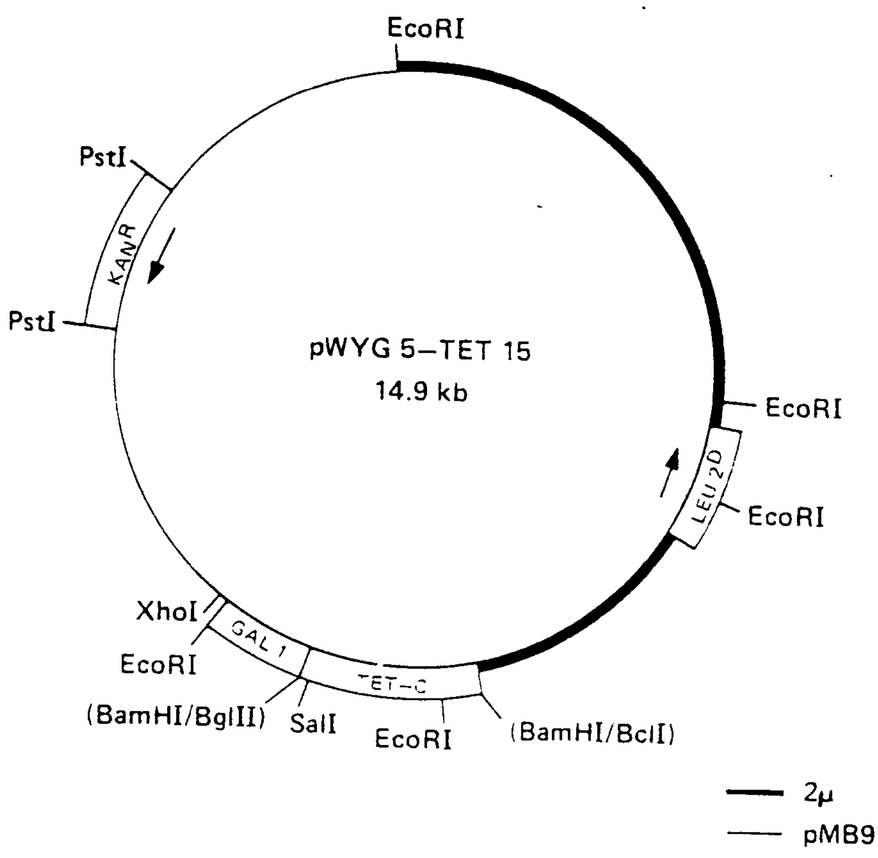
EcoRI
PstI
KANR
PstI
pWYG 5–TET 15
14.9 kb
EcoRI
LEU 2D
EcoRI
XhoI
GAL 1
EcoRI
(BamHI/BglII)
SalI
TET-C
EcoRI
(BamHI/BclI)
2μ
pMB9

FIG.13

(pWYG59-TET16)
←GAL 1→
G ATC CAA ACG
Bam HI

```
                    -30         -20         -10          1          10
Bam HI
GA TCT ACA TGA TAA AAA AAA CAG TTG AAT ATT CCC TCA AAA ATG AGA TTT CCT TCA ATT
←——GAL 7 (pWYG9-TET2)——————————————————————————————→ Met Arg Phe Pro Ser Ile

 20          30          40          50          60          70
TTT ACT GCA GTT TTA TTC GCA GCA TCC TCC GCA TTA GCT GCT CCA GTC AAC ACT ACA
Phe Thr Ala Val Leu Phe Ala Ala Ser Ser Ala Leu Ala Ala Pro Val Asn Thr Thr

    80          90         100         110         120         130
ACA GAA GAT GAA ACG GCA CAA ATT CCG GCT GAA GCT GTC ATC GGT TAC TCA GAT TTA
Thr Glu Asp Glu Thr Ala Gln Ile Pro Ala Glu Ala Val Ile Gly Tyr Ser Asp Leu

       140         150         160         170         180
GAA GGG GAT TTC GAT GTT GCT GTT TTG CCA TTT TCC AAC AGC ACA AAT AAC GGG TTA
Glu Gly Asp Phe Asp Val Ala Val Leu Pro Phe Ser Asn Ser Thr Asn Asn Gly Leu

190         200         210         220         230         240       Xho I
TTG TTT ATA AAT ACT ACT ATT GCC AGC ATT GCT GCT AAA GAA GAA GGG GTA TCT CTC
Leu Phe Ile Asn Thr Thr Ile Ala Ser Ile Ala Ala Lys Glu Glu Gly Val Ser Leu

   250         260         270
                      Nco I
GAG AAA AGA GAG GCT GAA GCC ATG G
Glu Lys Arg Glu Ala Glu Ala Met
```

(SEQ ID NO : 8)

FIG.14

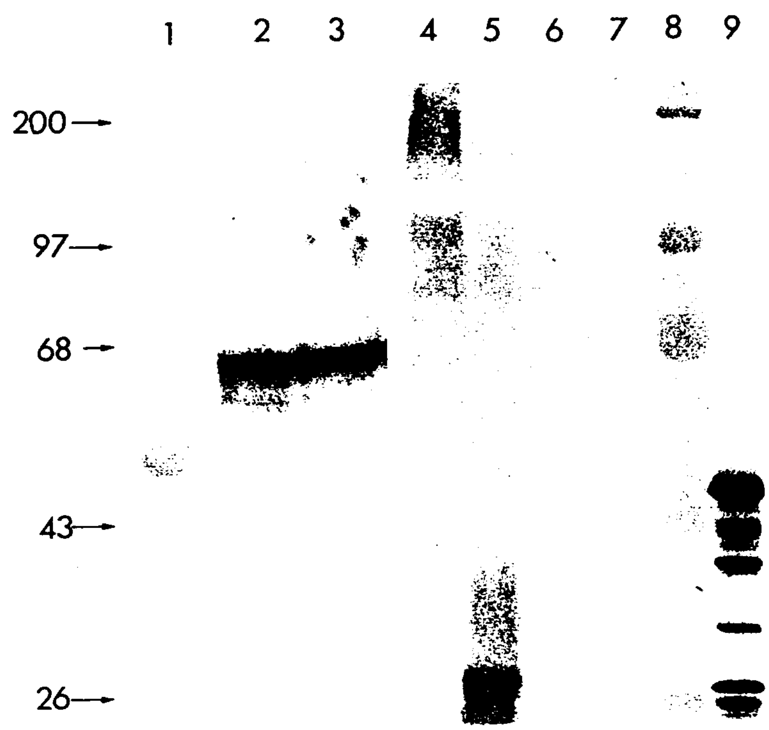

FIG.15

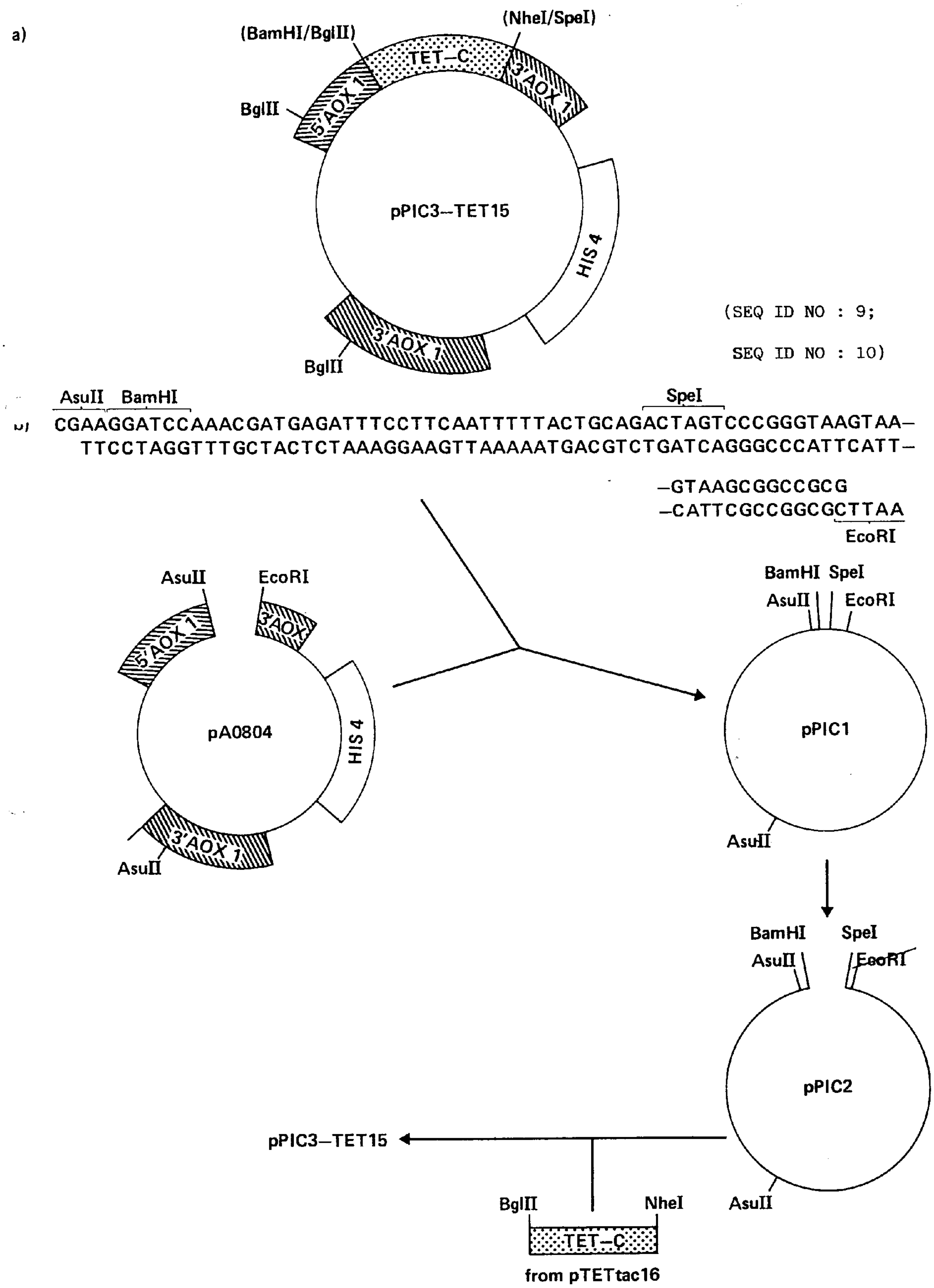
a)
(BamHI/BglII)
(NheI/SpeI)
TET–C
5'AOX 1
3'AOX 1
BglII
pPIC3–TET15
HIS 4
3'AOX 1
BglII
(SEQ ID NO : 9;
SEQ ID NO : 10)
b)
AsuII BamHI
SpeI
CGAAGGATCCAAACGATGAGATTTCCTTCAATTTTTACTGCAGACTAGTCCCGGGTAAGTAA–
TTCCTAGGTTTGCTACTCTAAAGGAAGTTAAAAATGACGTCTGATCAGGGCCCATTCATT–
–GTAAGCGGCCGCG
–CATTCGCCGGCGCTTAA
EcoRI
AsuII
EcoRI
5'AOX 1
3'AOX
pA0804
HIS 4
3'AOX 1
AsuII
BamHI SpeI
AsuII
EcoRI
pPIC1
AsuII
BamHI
SpeI
AsuII
EcoRI
pPIC2
AsuII
pPIC3–TET15
BglII
NheI
TET–C
from pTETtac16

FIG.16

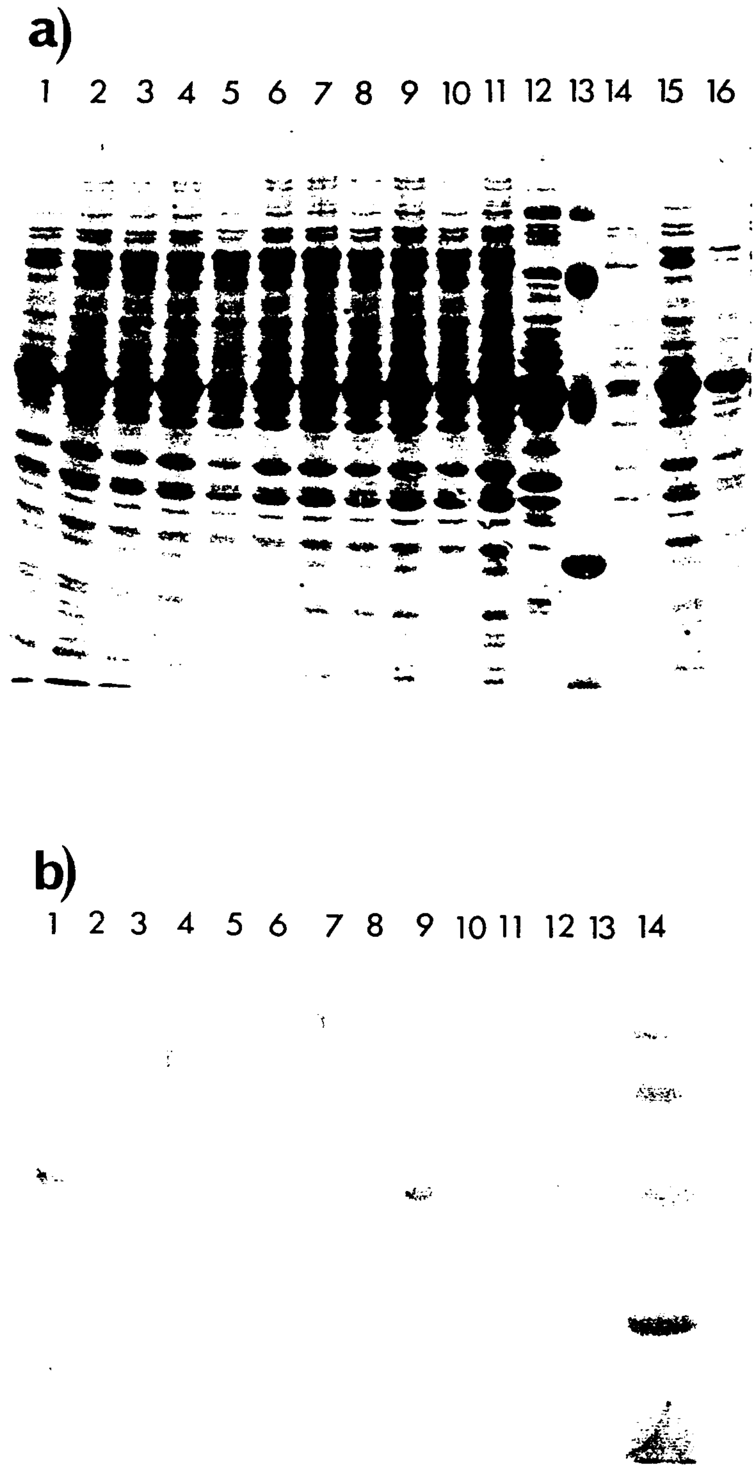

FIG.17